(19) Europäisches Patentamt
European Patent Office
Office européen des brevets

(11) Veröffentlichungsnummer: **0 225 496 B1**

(12) # EUROPÄISCHE PATENTSCHRIFT

(45) Veröffentlichungstag der Patentschrift: **20.05.92**

(51) Int. Cl.[5]: **C12P 19/04**, C08B 37/14

(21) Anmeldenummer: **86115584.4**

(22) Anmeldetag: **10.11.86**

Die Akte enthält technische Angaben, die nach dem Eingang der Anmeldung eingereicht wurden und die nicht in dieser Patentschrift enthalten sind.

(54) **Immunstimulierend wirkende Polysaccharide aus Zellkulturen von Echinacea purpurea (Linné) Moench und Echinacea angustifolia (De Vandolle), Verfahren zu ihrer Herstellung und sie enthaltende pharmazeutische Zubereitungen.**

(30) Priorität: **27.11.85 DE 3541945**

(43) Veröffentlichungstag der Anmeldung:
**16.06.87 Patentblatt 87/25**

(45) Bekanntmachung des Hinweises auf die Patenterteilung:
**20.05.92 Patentblatt 92/21**

(84) Benannte Vertragsstaaten:
**AT BE CH DE ES FR GB GR IT LI LU NL SE**

(56) Entgegenhaltungen:
**DE-A- 3 217 214**

**ARZNEIMITTEL FORSCHUNG, Band 35, Juli 1985, Seiten 1069-1075, Editio Cantor, Verlag für Medizin und Naturwissenschaften GmbH, Aulendorf, DE; H. WAGNER et al.: "Immunstimulierend wirkende Polysaccharide (Heteroglykane) aus höheren Pflanzen"**

(73) Patentinhaber: **LOMAPHARM Rudolf Lohmann GmbH KG Pharmazeutische Fabrik
Langes Feld 5
W-3254 Emmerthal 1(DE)**

Patentinhaber: **B.A.T. Cigaretten-Fabriken GmbH
Alsterufer 4
W-2000 Hamburg 36(DE)**

(72) Erfinder: **Wagner, Hildebert, Prof. Dr.
Nelkenweg
W-8211 Breitbrunn/Chiemsee(DE)**
Erfinder: **Zenk, Meinhart H., Prof.
Pfeivestlstrasse 17
W-8000 München 60(DE)**
Erfinder: **Ott, Holger
Bückebergstrasse
W-3254 Emmerthal 1(DE)**
Erfinder: **Brümmer, Bernd, Dr. Dipl.-Chem.
Neuer Luruperweg 33
W-2083 Halstenbek(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ARZNEIMITTEL FORSCHUNG, Band 34, Juni 1984, Seiten 659-661, Editio Cantor, Verlag für Medizin und Naturwissenschaften GmbH, Aulendorf, DE; H. WAGNER et al.: "Immunstimulierend wirkende Polysaccharide (Heteroglykane) aus höheren Pflanzen"

ADVANCES IN CARBOHYDRATE CHEMISTRY AND BIOCHEMISTRY, Band 42, 1984, Seiten 265,273-275,277-292,302-305,368-369, Academic Press, Inc., New York, US; P.M. DEY et al.: "Plant cell-walls"

H. BRAUN: "Heilpflanzen-Lexikon für Ärzte und Apotheker", zweite Auflage, 1974, Seite 75, Gustav Fischer Verlag, Stuttgart, DE;

(74) Vertreter: **Schwabe - Sandmair - Marx**
**Stuntzstrasse 16**
**W-8000 München 80(DE)**

## Beschreibung

Die vorliegende Erfindung betrifft neue, immunstimulierend wirkende Polysaccharide aus Zellkulturen von Echinacea purpurea (Linné) Moench, Echinacea angustifolia (De Vandolle), Verfahren zu ihrer Herstellung sowie diese Polysaccharide enthaltende Arzneimittel.

Die Immunstimulation als therapeutisches Konzept ist in der Medizin seit langem bekannt. Im allgemeinen versteht man darunter die Injektion von Substanzen, die selbst nur eine geringe bzw. überhaupt keine antigene Wirkung aufweisen, die jedoch in der Lage sind auf unspezifische oder auch auf spezifische Art und Weise die körpereigenen Abwehrmechanismen zu induzieren. Nach heutigen Erkenntnissen ist eine Vielzahl von Stoffen in der Lage. die Immunabwehr zu stimulieren, wobei insbesondere verschiedene Mineralien, z. B. $Al(OH)_3$, $MgSO_4$, Beryllium, pflanzliche Öle mit oder ohne Zusatz von Mykobakterien sowie eine Reihe von pflanzlichen Inhaltsstoffen zu nennen sind. In diesem Zusammenhang ist insbesonders die Stoffgruppe der Lectine zu erwähnen, die auf ihre immunstimulierende Wirkung intensiv untersucht wurde. Die Lectine (Phythämagglutine) sind pflanzliche Proteine oder Glykoproteine. Als weitere pflanzliche Inhaltsstoffe mit immunstimulierender Wirkung wurden Polysaccharide aus höheren Pflanzen, aus niederen und höheren Pilzen, aus Flechten und Algen isoliert und untersucht. Der gesamte Komplex der Immunstimulierung wird ausführlich beschrieben von z. B. Chedid, L., et al. Immunstimulation, Springer Verlag, Heidelberg, New York 1980; Heidelberger, M., Structure and Immunological Specificity of Polysaccarides. Fortschritte d. Chem. org. Naturst. 42, 288 (1982); Drews, H. Möglichkeiten der Immunstimulation. Swiss Pharma 2, 9, (49) (1980).

Die genaue Wirkungsweise der immunstimulierenden Substanzen konnte in den meisten Fällen bis heute nicht endgültig geklärt werden. Generell zeigen diese Substanzen unter anderem einen Einfluß auf die Proliferation der immunkompetenten Zellen, wobei sie jedoch keine Gedächtnisreaktion hinterlassen. Dies bedeutet, daß als Wirkungsziele für die immunstimulierenden Substanzen die Makrophagen und Granulozyten sowie T- und B-Lymphozyten im Vordergrund stehen. Die Wirkung der Immunstimulantien kann auf direktem oder indirektem Wege. z. B. über das Komplementsystem oder über Lymphozyten, über die Produktion von Interferon oder lysosomaler Enzyme (z. B. Lymphokine, colony stimulating-factor und andere), sowie über eine Steigerung der Makro- und Mikrophagozytose, erfolgen. Wegen der Verflechtung von unspezifischen und spezifischen Abwehrmechanismen ist dabei immer mit Kaskadeneffekten und der gleichzeitigen Beeinflussung mehrerer Abwehrsysteme zu rechnen.

In der Medizin ergeben sich als bevorzugte Anwendungsgebiete für die Immunstimulation vor allem die Therapie von Mischinfektionen, chronischen, persistierenden, chemotherapieresistenten, bakteriellen und viralen Infektionen, die Prophylaxe von opportunistischen Infektionen bei Risikopatienten, die Therapie der malignen Erkrankungen und in gewissem Umfang auch die Behandlung von Autoimmunerkrankungen. Gleichfalls können Immmunstimulantien bei einer Zytostatika-Therapie zur teilweisen Kompensation der damit verbundenen Immunsuppression eingesetzt werden.

Zur Isolierung von Polysacchariden aus pflanzlichen Ausgangsmaterialien werden in der Literatur eine Reihe von Extraktionsverfahren genannt. WHISTLER, R.L., SANNELLA, J.L., "Methods in Carbohydrate Chemistry", Herausg.: WHISTLER, R.L., BEMILLER, J.N., Vol. V, S. 34 - 36, Academic Press, New York 1965; TOMODA, M., SHIMADA, K., SAIZO, Y. und SUGI, M., CHem. Pharm. Bull. 28 (10), 2933 (1980).

So wird das vorliegende Pflanzenmaterial, je nach Polysaccharidtyp mit kaltem oder heißem Wasser, mit wässrigen Salzlösungen, verdünnten Säuren oder Laugen oder mit Dimethylsulfoxid extrahiert. Aus den so gewonnenen Lösungen erhält man die Polysaccharid-Rohfraktionen im allgemeinen durch Fällung mit Alkohol, über Komplexbildung mit Schwermetallsalzen oder quartären Ammoniumsalzen. Die Polysaccharidrohfraktionen werden anschließend durch Ionenaustausch-, Gelfiltration- und Bioaffinitäts-Chromatographie aufgetrennt.

Bei der Isolierung von Polysacchariden aus nativen pflanzlichen Ausgangsstoffen nach bekannten Verfahren wird man unausweichlich mit folgenden Nachteilen konfrontiert. Die bei der Extraktion mitanfallenden lipophilen Begleitstoffe, z. B. Chlorophyll erweisen sich als nur schwer oder erst nach langwieriger Extraktion mit organischen Lösungsmitteln abtrennbar. In Abhängigkeit von der Natur des nativen pflanzlichen Ausgangsmaterials erhält man bei verschiedenen Aufreinigungen ein und desselben Pflanzenmaterials immer wieder sowohl qualitativ als auch quantitativ unterschiedliche Zusammensetzungen der dargestellten Polysaccharidrohfraktionen. Ein weiterer schwerwiegender Nachteil besteht in der absolut erforderlichen Verwendung von alkalischen oder sauren Extraktionsmitteln, die bei der Isolierung von speziellen Polysaccharid-Typen unumgänglich sind. Durch die Verwendung dieser Extraktionsmittel werden die Primär-, Sekundär- oder Tertiärstruktur der Polysaccharide verändert und damit die immunstimulierende Wirkung beeinflußt.

Aufgabe der vorliegenden Erfindung ist es nun, Verfahren zur Gewinnung von Polysacchariden aus höheren Pflanzen aufzuzeigen, die die vorhin genannten Nachteile nicht aufweisen und eine einfache und effiziente Herstellung von Polysacchariden aus höheren Pflanzen ermöglichen.

Eine weitere Aufgabe der Erfindung besteht darin, pharmazeutische Formulierungen anzugeben, die die immunstimulierenden Mittel zur Verwendung in der Humantherapie enthalten.

Erfindungsgemäß werden pflanzliche Zellkulturen als Ausgangsmaterial verwendet, wobei die besonderen Vorteile der Verwendung von Zellkulturen zunächst darin liegt, daß die gewünschten Produkte in ständig gleichbleibender chemischer Zusammensetzung und Ausbeute erhalten werden. Darüber hinaus fallen die vorerwännten lipophilen Begleitstoffe bei Verwendung von Zellkulturen nicht oder nur zu einem sehr geringen Anteil an. Ein weiterer Vorteil von Zellkulturen besteht darin, daß die gewünschten Polysaccharide direkt in das die Zellen umgebende Nährmedium abgegeben werden, so daß die weitere Aufreinigung auf relativ einfachem Wege durchgeführt werden kann. Hier handelt es sich um eine ungewöhnliche Leistung der pflanzlichen Zellen, indem hohe Mengen an Polysacchariden in das Nährmedium ausgeschieden werden.

Ein weiterer Vorteil des erfindungsgemäßen Verfahren besteht darin, daß sich die Isolierungszeit der Polysaccharide verkürzt und die Behandlung des Ausgangsmaterials mit teilweise drastischen Extraktionsmitteln vermieden wird, so daß eine Artefaktbildung der gewünschten Produkte im Rahmen der Aufarbeitung großteils ausgeschlossen werden kann.

Im folgenden wird beispielhaft ein Verfahren zum Anlegen pflanzlicher Zellkulturen abgeleitet von Echinacea purpurea und anschließend ein Verfahren zur Gewinnung von immunstimulierenden Polysacchariden aus diesen Zellkulturen beschrieben.

Als Ausgangsmaterial zum Anlegen der Zellkulturen verwendet man sterile Keimlinge, Blatt-, Blüten-, Stengel- oder Wurzelteile aus Echinacea purpurea- oder Echinacea angustifolia-Beständen, die man auf festen Nährboden zunächst zur Kallusbildung anregt.

Zu diesem Zweck implantiert man Keimlinge, Blatt-; Blüten-, Stengel- und Wurzelteile von Echinacea purpurea in agarhaltiges Linsmaier-Skoog-Medium, (Linsmaier, E.M., F. Skoog, Physiol. Plant. 18, 100 (1965)) und inkubiert das Medium für 14 bis 28 Tage bei 24 °C unter Zusatz von Auxinen, z. B. 2,4-Dichlorphenoxyessigsäure. Qualitativ können die Auxine variiert werden. Wie in "Handbook of Plant Culture" angegeben (Editors: David, A. Evans, William R. Sharp, Philipp V. Ammirato und Aysuyuki Yamada, Vol. 1 , Macmillan Publishing Co. A Division of Macmillan. Inc. New York), können auch andere Nährmedien verwendet werden, um Kallus- und Zellkulturen herzustellen.

Nachdem sich der erste Kallus nach etwa 14 Tagen gebildet hat, wird dieser in ein flüssiges Nährmedium übertragen. Diese Zellkulturen sind Suspensionskulturen, wobei diese in 1 l oder 1,5 l Erlenmeyer-Kolben mit ca. 250 bzw. 1000 ml flüssigem Nährmedium gefüllt sind und bei 100 rpm geschüttelt werden. Nach etwa 14 Tagen Inkubation bei 22 - 28 °C filitriert man die Suspensionskultur ab, lyophilisiert den Zellrückstand zur Gewichtsbestimmung und verarbeitet das abfiltrierte Nährmedium weiter.

A) Isolierung der Polysaccharide aus dem Zellkulturmedium

Alle im folgenden beschriebenen Arbeiten werden, wenn nicht anders angegeben, bei 4 - 8°C ausgeführt.

a) Isolierung der Polysaccharid -Rohfraktion

Die Zellen und zellulären Bestandteile von insgesamt 20 l Zellsuspensionskulturen werden abfiltriert und der Rückstand mit 5 l destilliertem Wasser nachgewaschen.

Der zelluläre Rückstand wird zur Gewichtsbestimmung tiefgefroren und lyophilisiert. Dabei erhält man etwa 270 g lyophilisiertes Zellmaterial. Die vereinigten Filtrate (20 l) werden in Portionen zu je 3 l unter ständigem Rühren mit dem dreifachen Volumen Ethanol (95 %) versetzt. Die über Nacht gebildeten Niederschläge werden zunächst durch vorsichtiges Abdekantieren, anschließend durch Abzentrifugieren von den überstehenden Lösungen abgetrennt (10 000 Upm/30 min.). Die vereinigten Zentrifugate werden in 4 l destilliertem Wasser gelöst, unter Eiskühlung langsam mit 15 %-iger Trichloressigsäure zur Endkonzentration von 7,5 % versetzt und nach einer Stunde abzentrifugiert. Der gallertige Niederschlag wird verworfen, der klare Überstand portioniert und mit dem 3-fachen Vol. Ethanol (95 %) ausgefällt. Die gebildeten Niederschläge werden nach 12 Stunden abzentrifugiert. vereinigt und in 1/20 des ursprünglich eingesetzten Volumens Natriumacetatlösung (2%) aufgenommen. Die Lösung wird 8 Stunden bei 4 °C gerührt und anschließend filtriert. Das Filtrat wird nun mit dem 1-fachen Vol. Ethanol (95 %) versetzt, 12 Stunden stehengelassen, der gebildete Niederschlag abzentrifugiert, in 1/10 Vol. destilliertem Wasser aufgenommen, zwei Tage gegen entmineralisiertes Wasser dialysiert und anschließend lyophilisiert (1:1 Fällung).

Der Überstand der letzten 1 : 1 Fällung mit Ethanol wird mit dem 1,5-fachen Volumen Ethanol (95 %) versetzt und 48 Stunden stehengelassen. Anschließend wird der Niederschlag abzentrifugiert, in destilliertem Wasser gelöst, 48 Stunden dialysiert und gefriergetrocknet. Man erhält folgende Ausbeuten: 1 : 1 Fällung: 2 g, ca. 1 %, bezogen auf die gefriergetrockneten Zellbestandteile. 1 : 4 Fällung: 1 g, ca. 0.5%, bezogen auf die gefriergetrockneten Zellbestandteile.

Gefriergetrockneter Zellrückstand 270 g.

b) Nachfolgend wird zur Isolierung der Polysaccharid-Rohfraktion ein verkürztes Verfahren unter Weglassung der Trichloressigsäurefällung beschrieben.

Die Zellbestandteile von insgesamt 10 l Zellsuspensionskulturen werden abfiltriert und der Rückstand mit destilliertem Wasser nachgewaschen. Die zellulären Rückstände werden wiederum zur Gewichtsbestimmung lyophilisiert (ca. 120 g lyophilisiertes Zellmaterial) und die vereinigten Filtrate portionsweise mit dem dreifachen Volumen Ethanol (95 %) versetzt. Die gebildeten Niederschläge werden zunächst durch vorsichtiges Abdekantieren, anschließend durch Abzentrifugieren (10 000 Upm, 30 Min.) von den überstehenden Lösungen abgetrennt. Die vereinigten Zentrifugate werden in destilliertem Wasser gelöst, unlösliche Bestandteile abzentrifugiert (10 000 Upm, 30 Min.) und die überstehende Lösung mit dem einfachen Volumen Ethanol (95 %) versetzt. Die gebildeten Niederschläge werden nach 12 Stunden abzentrifugiert, anschließend das Zentrifugat in destilliertem Wasser gelöst, die Lösung 48 Stunden gegen entmineralisiertes Wasser dialysiert und dann lyophiliert. Der Überstand der 1 : 1 Fällung wird mit dem 1,5-fachen Vol. Ethanol (95 %) versetzt, der gebildete Niederschlag nach 48 Stunden abzentrifugiert (10 000 UpM, 30 Min.), dann in destilliertem Wasser gelöst, 48 Stunden gegen entmineralisiertes Wasser dialysiert und anschließend lyophilisiert (1:4 Fällung).

Man erhält folgende Ausbeuten:

1 : 1 Fällung: 1,5 g, ca. 1,2 %, bezogen auf das lyophilisierte Zellmaterial
1 : 4 Fällung: 0,7 g, ca. 0,5 %, bezogen auf das lyophilisierte Zellmaterial

Lyophilisierter Zellrückstand: 120 g.

B) Gewinnung eines neutralen Polysaccharides A und dreier weiterer Polysaccharide (B - D) aus der 1 : 1 Fällung

a) Vortrennung

Man beschickt eine Anionenaustauschsäule, beispielsweise eine DEAE-Sepharose® CL-6 B-Säule in Acetatform mit einer wässrigen Lösung der 1 : 1 Fällung. Im Wassereluat wird ein Neutralpolysaccharid A mit positiver optischer Drehung erhalten. Die Elution der sauren Polysaccharidfraktionen erfolgt durch einen NaCl-Gradienten von 0 - 0,75 M.

Bei einer NaCl-Konzentration von ca. 0,2 M wird ein Polysaccharid (B) mit negativer optischer Aktivität eluiert, bei einer Salzkonzentration von ca. 0,4 M werden zwei weitere rechtsdrehende Polysaccharidfraktionen (C, D), erhalten.

b) Reinigung des neutralen Polysaccharides A über Anionenaustauschchromatographie und Gelfiltration.

Zur Abtrennung noch vorhandener saurer Polysaccharidanteile wird die im Beispiel a) erhaltene Polysaccharidfraktion A zunächst einer Anionenaustauschchromatographie, bspw. über DEAE-Sepharose® CL-6 B und DEAE-Trisacryl® M, unterworfen. Beide Säulen werden mit $H_2O$ eluiert. Die auf diese Weise gereinigte Neutralfraktion A wird anschließend durch Gelfiltration über Sephacryl® S 400 weiter gereinigt. Dadurch gelingt es, niedermolekulare Bestandteile ohne optische Aktivitäten vom rechtsdrehenden Polysaccharid abzutrennen. Das gereinigte Polysaccharid A wird auf Einheitlichkeit geprüft und das Molekulargewicht bestimmt.

Man erhält folgende Ausbeute: ca. 0,07 % bezogen auf 100 g lyophilisiertes Zellmaterial.

C) Gewinnung der Polysaccharide E und F aus der 1 : 4 Fällung

a) Vortrennung

Durch Anionenaustauschchromatographie über DEAE-Sepharose® CL-6 B erhält man im Wassereluat ein Polysaccharid (E) mit positiver optischer Drehung. Nach Elution mit einer NaCl-Konzentration von ca. 0,2 M wird ein saures Polysaccharid mit negativer Drehung (Polyseccharid F) erhalten.

b) Reinigung der Polysaccharidfraktionen E und F

Polysaccharid E wird durch Gelfiltration, beispielsweise über Ultrogel® AcA mit 0,2 M NaCl-Lösung als Elutionsmittel gereinigt. Man findet das Polysaccharid E im Ausschlußvolumen der Säule, die Begleitsubstanzen mit niedrigeren Molekulargewicht ohne optische Aktivität findet man im Fraktionierbereich bzw. im Gesamtvolumen. Die Saure Polysaccharidfraktion F wird einer zweiten Anionenaustauschchromatographie, beispielsweise über DEAE-Trisacryl® M, unterworfen. Eluiert man mit reinem Wasser, so findet man in Eluat nur geringe Spuren. Das Polysaccharid F wird bei einer NaCl-

Konzentration von 0,2 M als relativ symmetrischer Peak eluiert. Beide Polysaccharide werden anschließend einer weiteren Gelfiltration über Sephacryl® S400 mit 0,2 M NaCl-Lösung als Elutionsmittel unterworfen.

Man erhält folgende Ausbeuten: Polysaccharid E 0,18 % Polysaccharid F 0,14 % jeweils bezogen auf 100 g lyophilisiertes Zellmaterial.

D) Großtechnisches Verfahren zur Isolierung der neutralen und sauren Polysaccharide gemäß Figur

1. Nachdem die Zellsuspension durch Filtrieren oder Zentrifugieren in den Zellrückstand und das zellfreie Nährmedium aufgetrennt und der Zellrückstand verworfen wurde, kann das zellfreie Nährmedium, das üblicherweise etwa 75 bis 85 Gew.-% des ursprünglichen Suspensionsvolumens ausmacht, bereits jetzt wie nachstehend unter 5. beschrieben, enteiweißt werden (Stufe 1.1). Dieser Schritt kann hier entfallen, muß dann jedoch bei Stufe 5 durchgeführt werden.

2. Resttrübungen durch Zellfragmente oder Eiweißausscheidungen werden durch Klärung im Durchlaufseparator, vorzugsweise in einem Kammer- oder Tellerseparator entfernt und die abgetrennten Feststoffe verworfen. Dieser Schritt kann unterbleiben, wenn die Enteiweißung bereits in Stufe 1. 1. nicht durchgeführt wird.

3. Kolloidal gelöste Bestandteile sowie hochmolekulare Verbindungen (Molekulargewichte > $10^6$ Dalton) werden durch Mikrofiltration entfernt (Stufe 3). Vorzugsweise verwendet man Polysulfonmembranen in Rohr- oder Kapillarmodulen mit einer Porengröße ≧ 0,1 µm. Wahlweise können hier auch Ultrafiltrationsmembranen mit Trenngrenzen > $10^5$ Dalton verwendet werden.

4. Zur Erzielung hoher Produktausbeuten wird das Retentat bei Erreichen eines vorgegebenen Minimalvolumens mit demineralisiertem Wasser diafiltriert. Permeat und Diafiltrat werden vereinigt, das Retentat wird verworfen.

Die vereinigten Permeate werden durch Ultrafiltration zunächst aufkonzentriert (Konzentrationsfaktoren zwischen 10 und 500, je nach Ausgangsvolumen und Anlagengröße) und anschließend bis zum Erreichen einer Leitfähigkeit von unter 100 µS/cm mit demineralisiertem Wasser diafiltriert. Das Permeat wird verworfen.

Die zur Aufkonzentration verwendeten Membranen sollten Trenngrenzen von 10 000 Dalton oder kleiner aufweisen. Vorzugsweise verwendet man Membranen aus Polysulfon oder Celluloseacetat in Rohr-, Kapillar- oder Spiralwickelmodulen.

Wahlweise kann anstelle der Diafiltration eine Entsalzung durch Dialyse oder Gelfiltration erfolgen.

5. Das konzentrierte Retentat wird, sofern nicht schon bei 1. 1. durchgeführt, enteiweißt. Hierfür kommen zwei Verfahrensalternativen zur Anwendung:

5. 1. Erhitzen auf Temperaturen von 90 °C bis 130 °C.

5. 2. Nach dem Abkühlen Filtrieren oder Zentrifugieren zur Abtrennung der Niederschläge

oder:

5. 1. Versetzen der Lösung mit Trichloressigsäure (TCA) bis zu einer Konzentration von 7,5 Gew.-%.

5. 2. Stehenlassen bei 4 °C für mindestens 12 Stunden.

5. 3. Abfiltrieren oder Zentrifugieren der Niederschläge.

5. 4. Dialyse der geklärten Lösung bis zu einer Leitfähigkeit < 100 µS/cm gegen demineralisiertes Wasser.

6. Das konzentrierte Retentat wird am schwachen Anionenaustauscher zunächst mit Wasser als Eluierungsmittel zur Gewinnung der neutralen Polysaccharidfraktion ( entsprechend Polysaccharidfraktion A) eluiert, anschließend mit einem Salzgradienten (Natriumacetat, NaCl, etc.) von 0 bis 4 molar zur Gewinnung der sauren Polysaccharidfraktionen (entsprechend Polysaccharidfraktion F) eluiert.

7. Die einzelnen Fraktionen können, sofern erforderlich, wie unter 5. beschrieben, enteiweißt werden.

8. Die Einzelfraktionen werden, wie unter 4. beschrieben, einer Ultra- und Diafiltration unterworfen.

9. Anschließend werden die Produkte durch Lyophilisation, Vakuumtrocknung oder Sprühtrocknung getrocknet.

Die erzielten Ausbeuten sind abhängig von der Qualität des jeweiligen Fermentationsansatzes und betragen für die einzelnen Fraktionen etwa 10 bis 100 ppm, bezogen auf das ursprüngliche Suspensionsvolumen.

E) Struktur der isolierten Polysaccaride

a) Polysaccarid A und E

1. Molekulargewichtsbestimmung

Die Molekulargewichtsbestimmung der Polysaccharide A und E erfolgte durch Gelchromatographie über Sephacryl® S 400 mit Wasser, bzw. 0,2 M NaCl-Lösung als Eluierungsmittel, sowie mittels HPLC.

Verwendetes HPLC-System:

a) $\mu$-Porasil-Gpc-60 + $\mu$-Bondagel E 125

b) $\mu$-Bondagel E 125 + 5-Bondagel E 500 Fa. Waters

Puffersysteme: 0,2 M bzw. 0,5 M Phosphatpuffer pH = 6,0

Vergleichssubstanzen: Dextran T 10, T 40, T 70, T 110, T 500, T 2000

Mit diesen Methoden wurde ein mittleres Molekulargewicht des Polysaccharids E mit 10 000 (Bereich: 5-15000) und des Polysaccharids A mit 25000 (Bereich 20-30000) bestimmt.

2. Struktur:

Beide Polysaccharide stellen Fuco-galakto-xylo-glucane dar, die aus Fucose, Galaktose, Xylose und Glucose im Verhältnis von 0,1 : 0,4 : 1 : 1,5 aufgebaut sind.

Die Hauptketten der Polysaccharide bestehen aus 1-4)-$\beta$-verknüpftenGlucoseeinheiten, die zu ca. 65 % in Position C-6 verzweigt sind.

Die Seitenketten sind relativ unterschiedlich zusammengesetzt und haben eine maximale Kettenlänge von 3 Zuckereinheiten. Im einfachsten Falle ist die terminale Xylose direkt am C-6 des Glucangerüstes gebunden. Daneben sind aber auch Galactose-(1-2)-Xylose, Fucose - (1-2) - Galactose -(1-2) - Xylose - und Galactose -(1-2) - Galaktose -(1-2) - Xylose Seitenketten vorhanden. Auf Grund der isolierten Oligosaccharide kann man annehmen, daß die Polysaccaride größtenteils aus sich wiederholenden Einheiten von Hepta- und Dekasacchariden aufgebaut sind.

Sowohl das Polysaccharid A als auch das Polysaccharid B sind zu ca. 8 % acetyliert.

Grundstruktur der Polysaccharide A und E

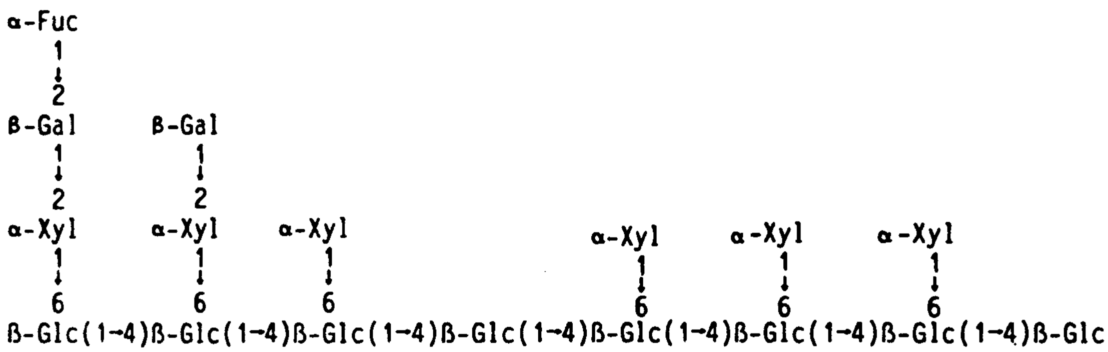

b) Grundstruktur des Polysaccharids F

1. Wie im Beispiel a) beschrieben, wurde das mittlere Molekulargewicht des Polysaccharids F je nach verwendetem Puffersystem mit 75.000 mit einem Bereich von 65000-85000 (0,5 M Phosphatpuffer pH 6,0) bzw. 110.000 mit einem Bereich von 100000 - 120000 (0,2 M Phosphatpuffer pH 6.0) bestimmt. Polysaccharid F stellt ein saures Arabinogalaktan dar. Das Grund

gerüst baut sich aus (1-3)-$\beta$-verknüpften Galaktose-Ketten auf, die über ein Rhamnogalakturonan miteinander verknüpft sind.

Jede zweite Galaktose-Einheit der (1-3)-$\beta$-Galaktan-Kette ist über das C-6-Atom mit (1-6)-$\beta$-Galaktose-Seitenketten verknüpft. Diese wiederum sind zu fast 70 % in Position C-3 mit terminaler Arabinose verknüpft.

Zusätzlich zu diesen Polysaccharid-Bestandteilen konnten noch längere, stark verzweigte (1-5)-$\alpha$-Arabinan-Ketten nachgewiesen werden. Dieser Arabinanteil kann am Arabinogalaktananteil oder am Rhamnogalakturonan gebunden sein. Struktur des Arabinogalaktanteils des Polysaccharids F

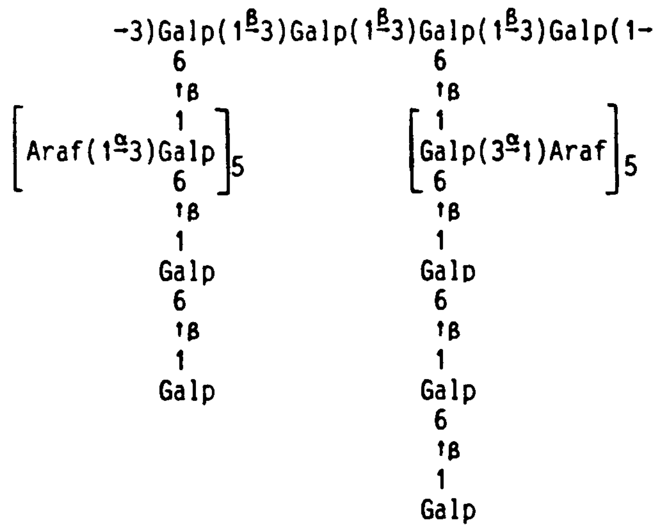

Grundstruktur für den Rhamnogalakturonan-Teil des Polysaccharids F

$$-2)Rha(1\rightarrow4)GalA(1\xrightarrow{\alpha}4)GalA(1\xrightarrow{\alpha}4)GalA(1\rightarrow2)Rha(1\rightarrow4)GalA(1\rightarrow$$

Grundstruktur des Arabinanteils des Polysaccharids F

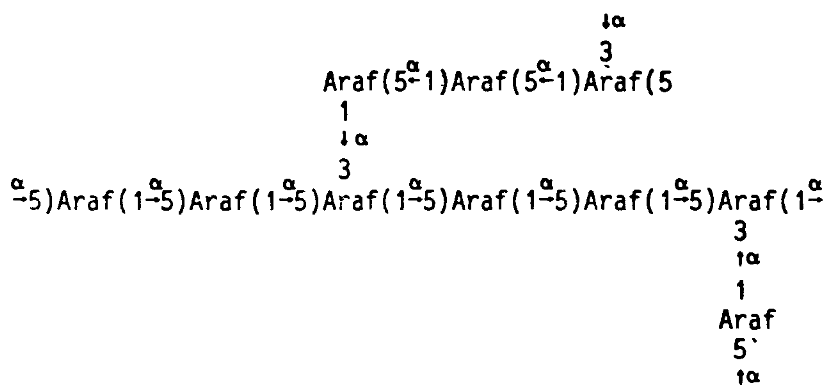

F) Pharmakologische Wirkung der isolierten Polysaccharide

Da es bislang keine spezifischen Testmethoden zur Überprüfung der immunstimulierenden Wirkung von Substanzen gibt, verwendet man bis heute primär solche in-vitro- und in-vivo-Verfahren, die den Einfluß von Verbindungen oder Pflanzenextrakten auf den Funktionszustand und die Leistungsfähigkeit des mononuklearen Systems, sowie die Stimulierbarkeit von T- und B-Lymphozyten zu messen gestatten.

a) Granulozyten-Test nach Brandt:

(Brandt, L., Scand. J. Haematol. (Suppl.) 2 (1967)

Im Granulozyten-Test nach Brandt wird im in-vitro Versuch die Zahl von phagozytierten Hefezellllen oder Bakterien durch eine Granulozytenfraktion, die aus Humanserum gewonnen wurde, unter dem

Mikroskop bestimmt. Man mißt die prozentuelle Steigerung der Phagozytose durch bestimmte Polysaccharidfraktionen.

Tabelle 1: Prozentuale Steigerung der Phagozytose durch einzelne Polysaccharidfraktionen im Granulozytentest nach BRANDT

| Polysaccharid-fraktionen | Phagozytosewerte in Prozent bei Konz. 1 | $10^{-1}$ | $10^{-2}$ | $10^{-3}$ | $10^{-4}$ | $10^{-5}$ mg/ml |
|---|---|---|---|---|---|---|
| aus dem Zell-kulturmedium: | | | | | | |
| 1:1 Fällung | - | 21,5 % | 27,8 % | 0 % | - | - |
| 1:4 Fällung | - | 15,4 % | 18,3 % | 13,5 % | - | - |
| PS* A | 17,6 % | 21,3 % | 28,1 % | 20,1 % | 9,3 % | - |
| PS E | - | 10,8 % | -4 % | -1,5 % | - | - |
| PS F | - | 0,6 % | 3 % | 10,8 % | - | - |

*PS = Polysaccharid

b) ein weiterer Test zur Bestimmung der Wirkung von immunstimulierenden Substanzen ist die sogenannte Carbon-Clearance-Methode. Bei dieser Methode wird spektrophotometrisch die Eliminationsrate von Kohlepartikel aus dem Blut von Tieren gemessen. Diese Eliminationsrate stellt ein Maß für die Phagozytoseaktivität dar (Biozzi, G., Benacerraf, B., Halpern, B. N., Br. J. Exp. Pathol. 34, 441 (1953)).

In der folgenden Tabelle 2 ist der Einfluß der Polysaccaridfraktionen aus der Zellkultur auf die Carbon-Clearance bei Mäusen dargestellt.

Tabelle 2: Einfluß von Polysaccharidfraktionen aus der Zellkultur auf die Carbon-Clearance bei Mäusen

Verabreichung von jeweils einmal 10 mg Substanz/kg Maus

| Polysaccharidfraktion | $K_{Substanz}/K_{Kontrolle}$* | DSK-Wert |
|---|---|---|
| 1 : 1 Fällung | 1,77 | 2 |
| PS A | 1,6 | 2 |
| PS E | 1,2 | 1 |
| PS F | 1,4 | 1 |

* Die Regressionskoeffizienten wurden unter Berücksichtigung des Leber-, Milz- und Körpergewichtes der jeweiligen Maus nach DREWS (115) korrigiert.

In den immunologischen Untersuchungen zeigte das 1 : 1 Fällungsprodukt aus dem Nährmedium im Granulozytentest deutlich höhere Phagozytoseraten als das 1 : 4 Fällungsprodukt. Derselbe Aktivitätsunterschied konnte auch bei den aus dem 1 : 1 bzw. 1 : 4 Fällungsprodukt isolierten Einzelkomponenten festgestellt werden.

Polysaccharide A

Das aus der 1 : 1 Fällung mit Ethanol isolierte Polysaccharid A zeigte im Gegensatz zu Polysaccharid E aus der 1 : 4 Fällung, übereinstimmend im Granulozyten- und Carbon-Clearance-Test, deutlich höhere immunstimulierende Aktivität.

Polysaccharid F

Das Polysaccharid F erwies sich wie andere geprüfte Arabinogalaktane in den verwendeten Testsystemen als nur mäßig aktiv, zeigte dagegen im TNF-Test (Tumor-Nekrosis-Faktor-Test) eine deutliche Wirkung.

c) Makrophagenaktivierung zu direkten Zytotoxizität gegen Tumorzellen, bestimmt nach

Stimpl, M., Proksch, A., Wagner, H. und Lohmann-Matthes, M.L., Infection and Immunity 46, 845 (1984).

Polysaccharid A aktivierte in diesem Versuch 2 x $10^5$ Maus-Peritonealmakrophagen zu vollständiger Zytotoxizität gegen P 815 Tumorzellen bis zu einer Verdünnung von 6 bis 50 $\mu$g pro 2 x $10^5$ Makrophagen.

Die erfindungsgemäßen Polysaccharide können entweder getrennt als Reinsubstanz oder in Form von pharmazeutischen Zubereitungen verabreicht werden, wenngleich es im allgemeinen bevorzugt ist, die Verbindungen in einer Kombination zu verabreichen. Vorzugsweise liegt die Arzneimittelkombination in Form einer Formulierung vor, die

(1) die erfindungsgemäßen Polysaccharide entweder allein oder in Kombination miteinander enthält und

(2) eines oder mehrere dafür geeignete Bindemittel, Trägermaterialien und/oder weitere Hilfsstoffe und

(3) gegebenenfalls weitere therapeutische Wirkstoffe aufweist.

Die Trägermaterialien, Bindemittel und/oder Hilfsstoffe müssen pharmazeutische und pharmakologisch verträglich sein, so daß sie mit den anderen Bestandteilen der Formulierung bzw. Zubereitung vereinbar sind und keine nachteilige Wirkung auf den behandelten Organismus ausüben.

Die Formulierungen schließen jene ein, die für die orale oder parenterale (einschließlich subkutane, intradermale, intramuskuläre und intravenöse) Verabreichung geeignet sind, wenngleich der am besten geeignete Verabreichungsweg von dem Zustand des Patienten abhängt.

Die Formulierungen können als Einzeldosierung vorliegen. Die Herstellung der Formulierungen erfolgt unter Anwendung von an sich auf dem Gebiet der Pharmazie bekannten Methoden. Alle Methoden schließen den Schritt ein, die erfindungsgemäßen Polysaccharide (d. h. die Wirkstoffe) mit dem Trägermaterial, Bindemittel und/oder Hilfsstoff, wobei letztere einen zusätzlichen Bestandteil darstellen, zu vermischen bzw. zu vereinigen. Im allgemeinen werden die Formulierungen dadurch hergestellt, daß man die Wirkstoffe mit den flüssigen Trägermaterialien oder den feinteiligen Trägermaterialien oder beiden innig vermischt und dann erforderlichenfalls das erhaltene Produkt in die gewünschte Verabreichungsform bringt. Die erfindungsgemäßen Formulierungen, die für die orale Verabreichung geeignet sind, können in Form von diskreten Einheiten, wie Kapseln, Cachets oder Tabletten, die jeweils eine vorbestimmte Menge des erfindungsgemäßen Wirkstoffes enthalten, sowie in Form von Pulvern oder Granulaten, in Form einer Lösung oder einer Suspension in einer wässrigen oder nicht wässrigen Flüssigkeit, oder in Form einer flüssigen Öl-in-Wasser-Emulsion oder einer flüssigen Wasser-in-Öl-Emulsion vorliegen.

Die Wirkstoffe können auch in Form eines Bolus oder einer Paste vorliegen.

Die Tabletten kann man durch Pressen oder Verformen herstellen, wobei man gegebenenfalls eines oder mehrere der üblichen Hilfsmittel zugibt.

Die für die parenterale Verabreichung geeigneten Formulierungen schließen sterile Injektionslösungen in wässrigen oder nicht wässrigen Medien, die Antioxidantien, Puffer, Bakteristostatika und gelöste Materialien, die die Formulierung im Hinblick auf den menschlichen Organismus isotonisch machen, ein. Weiter können die erfindungsgemäßen Polysaccharide in Form von sterilen Suspensionen in wässrigen oder nicht wässrigen Medien, die Suspensionsmittel und Verdickungsmittel enthalten können, vorliegen. Die Formulierungen können als Einzel- oder Mehrfach-Dosis vorliegen, beispielsweise in Form von Ampullen oder dicht verschlossenen Fläschchen und können auch in lyophilisierter Form gelagert werden, so daß es lediglich erforderlich ist, bei notwendiger Verabreichung steriles flüssiges Trägermaterial, beispielsweise für Injektionszwecke geeignetes Wasser, unmittelbar vor Verwendung zuzugeben. Die unmittelbar vor der Verabreichung bereiteten injektions lösungen und Suspensionen können aus sterilen Pulver, Granulaten und Tabletten der oben beschiebenen Art bereitet werden.

Die erfindungsgemäßen Zubereitungen können neben den vorhin genannten Bestandteilen andere für die in Rede stehenden Formulierungen geeignete Bestandteile enthalten. So können beispielsweise die auf oralem Weg zu verabreichenden pharmazeutischen Zubereitungen Aromastoffe enthalten.

Die zur Applikation geeigneten Mengen der Wirkstoffe variieren in Abhängigkeit vom jeweiligen Therapiegebiet. Im allgemeinen beträgt die Wirkstoffkonzentration einer Einzeldosisformulierung 5 bis 95 % der gesamten Formulierung. Dies entspricht bei einer einmaligen Applikation 1 bis 50 mg pro kg Körpergewicht. Diese Dosierung kann jedoch in Abhängigkeit der eingesetzten Applikation, des Patientenzustandes und des Therapiegebietes in breiten Grenzen variiert werden.

**Patentansprüche**

**Patentansprüche für folgende Vertragsstaaten : DE, NL, GB, CH, LI, FR, LU, BE, SE, IT**

**1.** Polysaccharide mit immunstimulierender Wirkung, **dadurch gekennzeichnet,** daß sie ein mittleres Molekulargewicht von 10 000 mit einem Bereich von 5 000 - 15 000 haben und folgende Grundstruktur aufweisen:

```
α-Fuc
  1
  ↓
  2
ß-Gal      ß-Gal
  1          1
  ↓          ↓
  2          2
α-Xyl      α-Xyl      α-Xyl                     α-Xyl      α-Xyl      α-Xyl
  1          1          1                         1          1          1
  ↓          ↓          ↓                         ↓          ↓          ↓
  6          6          6                         6          6          6
ß-Glc(1→4)ß-Glc(1→4)ß-Glc(1→4)ß-Glc(1→4)ß-Glc(1→4)ß-Glc(1→4)ß-Glc(1→4)ß-Glc
```

**2.** Polysaccharide mit immunstimulierender Wirkung, **dadurch gekennzeichnet,** daß sie ein mittleres Molekulargewicht von 25 000 mit einem Bereich von 20 000 - 30 000 haben und folgende Grundstruktur aufweisen:

```
α-Fuc
  1
  ↓
  2
ß-Gal      ß-Gal
  1          1
  ↓          ↓
  2          2
α-Xyl      α-Xyl      α-Xyl                     α-Xyl      α-Xyl      α-Xyl
  1          1          1                         1          1          1
  ↓          ↓          ↓                         ↓          ↓          ↓
  6          6          6                         6          6          6
ß-Glc(1→4)ß-Glc(1→4)ß-Glc(1→4)ß-Glc(1→4)ß-Glc(1→4)ß-Glc(1→4)ß-Glc(1→4)ß-Glc
```

**3.** Polysaccharide mit immunstimulierender Wirkung, dadurch gekennzeichnet, daß ihr mittleres Molekulargewicht in 0,2 M Phosphatpuffer 110 000, mit einem Bereich von 100 000 - 120 000 beträgt und ihre Grundstruktur im wesentlichen aus folgenden Teilen aufgebaut ist:

a) ein Arabinogalaktanteil der Formel:

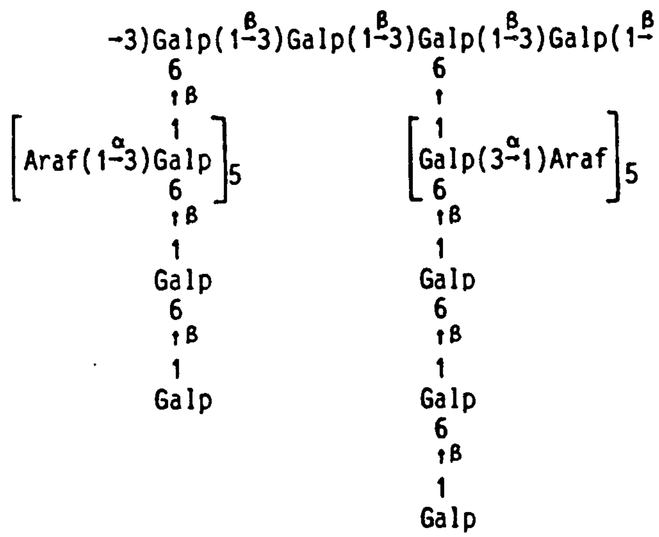

b) ein Rhamnogalakturonan-Anteil der Formel:

$$\rightarrow 2)Rha(1\rightarrow 4)GalA(1\xrightarrow{\alpha}4)GalA(1\xrightarrow{\alpha}4)GalA(1\xrightarrow{\alpha}2)Rha(1\rightarrow 4)GalA(1\xrightarrow{\alpha}$$

c) ein Arabinan-Anteil der Formel:

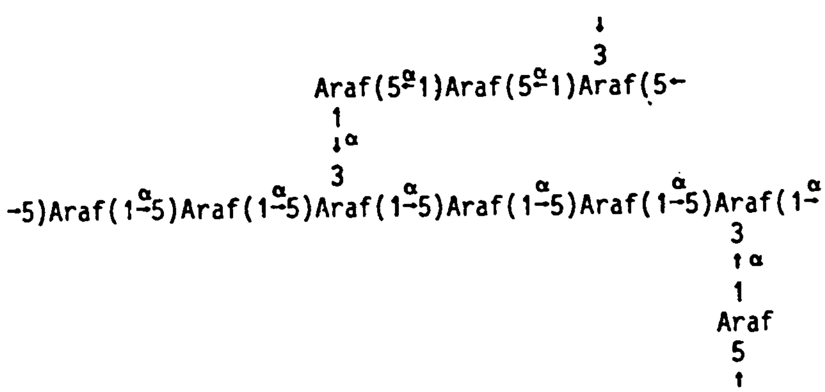

**4.** Polysaccharide nach Anspruch 1 bis 3, **dadurch gekennzeichnet,** daß sie aus pflanzlichen Zellkulturen von Echinacea purpurea (Linné) Moench und Echinacea angustifolia (De Vandolle) isoliert wurden.

**5.** Verfahren zur Herstellung der Polysaccharide nach Anspruch 1 bis 4, **dadurch gekennzeichnet,** daß man

a) sterilisierte Keimlinge, Blatt-, Blüten-, Stengel- und Wurzelteile pflanzlichen Ausgangsmaterials von Echinacea purpurea (Linné) und Echinacea angustifolia (De Vandolle) als Ausgangsmaterial verwendet,

b) sie zur Kallusbildung in ein agarhaltiges Nährmedium überführt,

c) aus dem gebildeten Kallus Primärzellen für eine Zellsuspension nach an sich bekannten Methoden gewinnt,

d) die Zellsuspensionskulturen nach 12 - 20, vorzugsweise 14 Tagen bei 22 - 28 °C abfiltriert, die Zellrückstände zur Gewichtsbestimmung lyophilisiert,

e) den Zellkultur-Überstand mit dem 2 - 4-fachen, vorzugsweise 3-fachen Volumen Ethanol versetzt,

f) den gebildeten Niederschlag durch Zentrifugation abtrennt, anschließend in destilliertem Wasser aufnimmt,
g) mit 15 %-iger Trichloressigsäure zu einer Endkonzentration von 7,5 % versetzt, stehen läßt, anschließend zentrifugiert,
h) den nach der Zentrifugation erhaltenen Überstand mit dem 3 - 6-fachen, vorzugsweise 4-fachen Volumen Ethanol versetzt, den gebildeten Niederschlag nach 12 Stunden Standzeit zentrifugiert, anschließend in 2 %-iger Natriumacetatlösung aufnimmt, diese Lösung für 8 Stunden rührt und anschließend filtriert,
i) das Filtrat mit dem 0,5 - 1,5-fachen, vorzugsweise 1-fachen Volumen Ethanol versetzt, 12 Stunden stehen läßt, den gebildeten Niederschlag abzentrifugiert und in 1/8 bis 1/10 des ursprünglichen Volumens destilliertem Wasser aufnimmt, 2 Tage gegen entmineralisiertes Wasser dialysiert und anschließend lyophilisiert,
j) den Überstand der ersten Alkohol-Fällung mit dem 1,5 - 2,5-fachen, vorzugsweise 2-fachen Volumen Ethanol versetzt, die Lösung für 48 Stunden stehen läßt, den Niederschlag abzentrifugiert, in destilliertem Wasser löst, die Lösung 48 Stunden gegen entmineralisiertes Wasser dialysiert und anschließend lyophilisiert.

**6.** Verfahren zur Herstellung der Polysaccharide nach Anspruch 1 bis 4, **dadurch gekennzeichnet,** daß man
a) sterilisierte Keimlinge, Blatt-, Blüten-, Stengel- und Wurzelteile pflanzlichen Ausgangsmaterials von Echinacea purpurea (Linné) und Echinacea angustifolia (De Vandolle) als Ausgangsmaterial verwendet,
b) sie zur Kallusbildung in ein agarhaltiges Nährmedium überführt,
c) aus dem gebildeten Kallus Primärzellen für eine Zellsuspension nach an sich bekannten Methoden gewinnt,
d) die Zellsuspensionskultur abfiltriert,
e) das Filtrat mit dem 2 - 4-fachen, vorzugsweise dreifachen, Volumen Ethanol versetzt, den gebildeten Niederschlag zunächst durch Abdekantieren, anschließend durch Abzentrifugieren abtrennt,
f) das Zentrifugat in Wasser löst, unlösliche Bestandteile abzentrifugiert, den Überstand mit dem 0,5 - 1,5-fachen, vorzugsweise 1-fachen Volumen Ethanol versetzt und für 48 Stunden stehen läßt,
g) den gebildeten Niederschlag abzentrifugiert, anschließend in destilliertem Wasser löst, die Lösung 48 Stunden gegen demineralisiertes Wasser dialysiert und anschließend lyophilisiert,
h) den Überstand der ersten Ethanol-Fällung mit dem 1,5 - 2,5-fachen, vorzugwsweise 2-fachen Volumen Ethanol versetzt, 12 Stunden stehen läßt, den gebildeten Niederschlag abzentrifugiert, anschließend in destilliertem Wasser löst, 48 Stunden gegen entmineralisiertes Wasser dialysiert und anschließend lyophilisiert.

**7.** Verfahren nach Anspruch 5 oder 6 zur Feinreinigung der Polysaccharide, **dadurch gekennzeichnet,** daß man die Polysaccharide aus der 1 : 1 Fällung mit Ethanol folgenden Reinigungsschritten unterwirft:
a) einer Anionenaustauschchromatographie in wässriger Lösung,
b) mit $H_2O$ als Elutionsmittel eine erste Polysaccharidfraktion isoliert und
c) weitere, saure Polysaccharide mittels eines NaCl-Gradienten von 0 bis 0,75 M eluiert.

**8.** Verfahren nach Anspruch 5 oder 6, **dadurch gekennzeichnet,** daß man die neutralen Polysaccharide aus der 1 : 1 Fällung mittels folgender Reinigungsschritte isoliert:
a) die Polysaccharide zunächst einer Anionenaustauschchromatographie mit $H_2O$ als Elutionsmittel, unterwirft,
b) die vereinigten polysaccharidhaltigen Fraktionen einer weiteren Anionenaustauschchromatographie unterwirft, mit $H_2O$ eluiert und
c) die vereinigten Polysaccharidfraktionen durch Gelchromatographie unter Verwendung von $H_2O$ oder 0,2 M NaCl als Eluierungsmittel weiter reinigt.

**9.** Verfahren nach Anspruch 5 oder 6, **dadurch gekennzeichnet,** daß man die Polysaccharide aus der 1 : 4 Fällung durch folgende Reinigungsschritte isoliert:
a) zunächst durch Anionenaustauschchromatographie das neutrale Polysaccharid unter Verwendung von $H_2O$ als Eluierungsmittel abtrennt, anschließend

b) unter Verwendung eines NaCl Konzentrationsgradienten von 0 bis 0,4 M das saure Polysaccharid eluiert, weiter

c) das neutrale Polysaccharid durch Gelfiltrationschromatographie unter Verwendung von 0,2 M NaCl oder $H_2O$ als Eluierungsmittel weiter reinigt, und

d) das saure Polysaccharid einer weiteren Anionenaustauschchromatographie unter Verwendung von zunächst $H_2O$ um zufällig vorhandene neutrale Polysaccharide abzutrennen, dann das saure Polysaccharid unter Verwendung eines NaCl-Gradienten von 0 bis 0,4 M eluiert, und

e) beide Polysaccharide einer weiteren Gelfiltrationschromatographie unter Verwendung von 0,2 M NaCl-Lösung als Elutionsmittel unterwirft.

**10.** Verfahren nach Anspruch 5 oder 6, **dadurch gekennzeichnet,** daß man zur Ausfällung der Polysaccharide Aceton, Ammoniumsulfat, Cetyltrimethylammoniumbromid, $Ca_2Cl_2$ oder Fehling'sche Lösung verwendet.

**11.** Verfahren zur Herstellung der Polysaccharide nach Anspruch 1 bis 4, **dadurch gekennzeichnet,** daß man

a) sterilisierte Keimlinge, Blatt-, Blüten-, Stengel- und Wurzelteile pflanzlichen Ausgangsmaterials von Echinacea purpurea und Echinacea angustifolia als Ausgangsmaterial verwendet, diese zur Kallusbildung in ein agarhaltiges Nährmedium überführt, aus dem gebildeten Kallus Primärzellen für eine Zellsuspension nach an sich bekannten Methoden gewinnt, die Zellsuspensionskulturen nach 12 - 20, vorzugsweise 14 Tagen bei 22 - 28° C abfiltriert, die Zellrückstände verwirft,

b) Resttrübungen des Nährmediums durch Zellfragmente oder Eiweißausscheidungen durch Klärung im Durchlaufseparator, vorzugsweise in einem Kammer- oder Tellerseparator, entfernt und die abgetrennten Feststoffe verwirft,

c) kolloidal gelöste Bestandteile sowie hochmolekulare Verbindungen (Molekulargewichte > $10^6$ Dalton) durch Mikrofiltration entfernt, wobei vorzugsweise Polysulfonmembranen in Rohr- oder Kapillarmodulen mit einer Porengröße ≧ 0,1 $\mu$m oder Ultrafiltrationsmembranen mit Trenngrenzen > $10^5$ Dalton verwendet werden,

d) das Retentat bei Erreichen eines vorgegebenen Minimalvolumens mit demineralisiertem Wasser diafiltriert, Permeat und Diafiltrat vereinigt, das Retentat verwirft und die vereinigten Permeate durch Ultrafiltration aufkonzentriert (Konzentrationsfaktoren zwischen 10 und 500, je nach Ausgangsvolumen und Anlagengroße), anschließend bis zum Erreichen einer Leitfähigkeit von unter 100 $\mu$S/cm mit demineralisiertem Wasser diafiltriert und das Permeat verwirft,

e) das konzentrierte Retentat durch

e.1 Erhitzen auf Temperaturen von 90° C bis 130° C und

e.2 nach dem Abkühlen zur Abtrennung der Niederschläge filtriert oder zentrifugiert oder dadurch enteiweißt daß man

e.1 die Lösung mit Trichloressigsäure (TCA) bis zu einer Konzentration von 7,5 Gew.% versetzt,

e.2 bei 4° C mindestens 12 Stunden stehen läßt,

e.3 die Niederschläge abfiltriert oder zentrifugiert,

e.4 die geklärte Lösung bis zu einer Leitfähigkeit < 100 $\mu$S/cm gegen demineralisiertes Wasser dialysiert,

f) das konzentrierte Retentat am schwachen Anionenaustauscher zunächst mit Wasser als Eluierungsmittel zur Gewinnung der neutralen Polysaccharidfraktion (entsprechend Polysaccharidfraktion A) und anschließend mit einem Salzgradienten (Natriumacetat, NaCl, etc.) von 0 bis 4 molar zur Gewinnung der sauren Polysaccharidfraktionen (entsprechend Polysaccharidfraktion F) eluiert,

g) die Einzelfraktionen gegebenenfalls wie oben unter e) enteiweißt,

h) die Einzelfraktionen, wie unter d), einer Ultra- und Diafiltration unterwirft und

i) anschließend die Produkte durch Lyophilisation, Vakuumtrocknung oder Sprühtrocknung trocknet.

**12.** Verfahren nach Anspruch 11, **dadurch gekennzeichnet,** daß man die Enteiweißung gemäß Stufe e) bereits im Anschluß an die Stufe a) durchführt.

**13.** Verfahren nach Anspruch 11 bis 12, **dadurch gekennzeichnet,** daß man auf Stufe d) Membranen aus Polysulfon oder Celluloseacetat in Rohr-, Kapillar- oder Spiralwickelmodulen verwendet, die Trenngrenzen von 10 000 Dalton oder weniger aufweisen.

**14.** Verfahren nach Anspruch 11 bis 13, **dadurch gekennzeichnet,** daß man auf Stufe d) anstelle der Dialfiltration eine Entsalzung durch Dialyse oder Gelfiltration durchführt.

**15.** Pharmazeutische Zubereitung, **dadurch gekennzeichnet,** daß die Polysaccharide nach Anspruch 1 bis 4 neben üblichen, pharmazeutisch verträglichen Träger- und Hilfsstoffen enthalten sind.

**Patentansprüche für folgende Vertragsstaaten : AT, ES, GR**

**1.** Verfahren zur Herstellung von Polysacchariden aus pflanzlichen Zellkulturen von Echinacea purpurea (Linné) Moench und Echinacea angustifolia (De Vandolle), die folgende Grundstrukturen (I), (II) und (III) aufweisen:

(I): ein mittleres Molekulargewicht von 10 000 mit einem Bereich von 5 000 - 15 000 und folgende Grundstruktur:

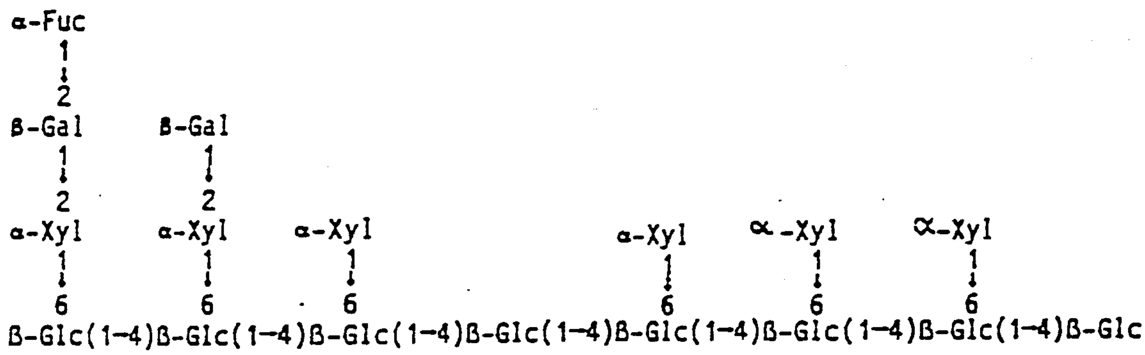

(II): ein mittleres Molekulargewicht von 25 000 mit einem Bereich von 20 000 - 30 000 und folgende Grundstruktur:

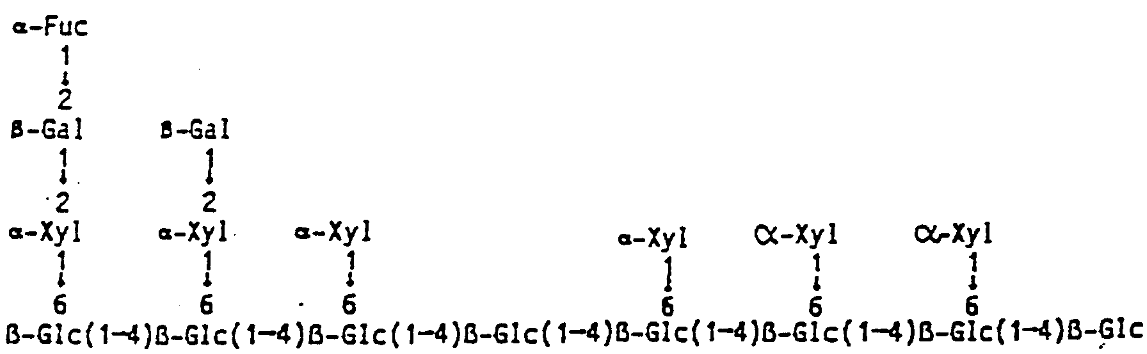

(III): ein mittleres Molekulargewicht in 0,2 M Phosphatpuffer von 110 000 mit einem Bereich von 100 000 - 120 000 und einer Grundstruktur, die im wesentlichen aus folgenden Teilen aufgebaut ist:

a) ein Arabinogalaktanteil der Formel:

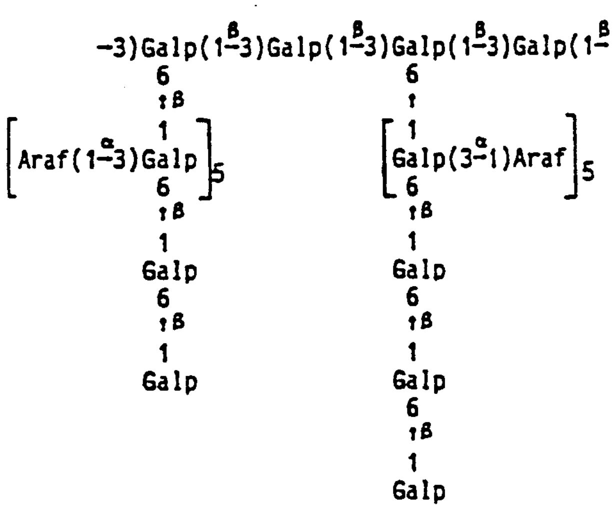

b) ein Rhamnogalakturonan-Anteil der Formel:

$$-2)Rha(1\rightarrow4)GalA(1\xrightarrow{\alpha}4)GalA(1\xrightarrow{\alpha}4)GalA(1\xrightarrow{\alpha}2)Rha(1\rightarrow4)GalA(1\xrightarrow{\alpha}$$

c) ein Arabinan-Anteil der Formel:

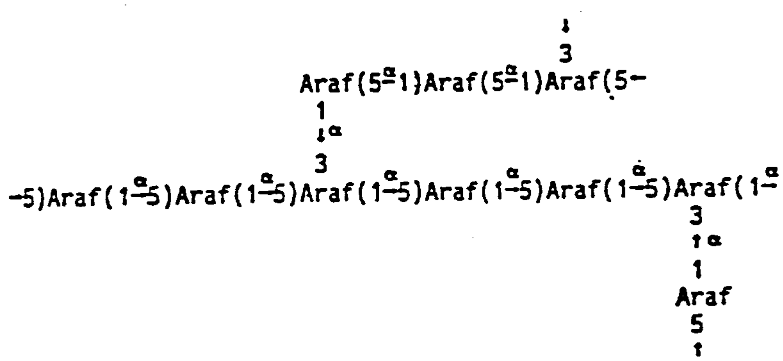

**dadurch gekennzeichnet,** daß man

a) sterilisierte Keimlinge, Blatt-, Blüten-, Stengel- und Wurzelteile pflanzlichen Ausgangsmaterials von Echinacea purpurea (Linné) und Echinacea angustifolia (De Vandolle) als Ausgangsmaterial verwendet,

b) sie zur Kallusbildung in ein agarhaltiges Nährmedium überführt,

c) aus dem gebildeten Kallus Primärzellen für eine Zellsuspension nach an sich bekannten Methoden gewinnt,

d) die Zellsuspensionskulturen nach 12 - 20, vorzugsweise 14 Tagen bei 22 - 28 °C abfiltriert, die Zellrückstände zur Gewichtsbestimmung lyophilisiert,

e) den Zellkultur-Überstand mit dem 2 - 4-fachen, vorzugsweise 3-fachen Volumen Ethanol versetzt,

f) den gebildeten Niederschlag durch Zentrifugation abtrennt, anschließend in destilliertem Wasser aufnimmt,

g) mit 15 %-iger Trichloressigsäure zu einer Endkonzentration von 7,5 % versetzt, stehen läßt, anschließend zentrifugiert,

h) den nach der Zentrifugation erhaltenen Überstand mit dem 3 - 6-fachen, vorzugsweise 4-fachen Volumen Ethanol versetzt, den gebildeten Niederschlag nach 12 Stunden Standzeit zentrifugiert, anschließend in 2 %-iger Natriumacetatlösung aufnimmt, diese Lösung für 8 Stunden rührt und anschließend filtriert,

i) das Filtrat mit dem 0,5-1,5-fachen, vorzugsweise 1-fachen Volumen Ethanol versetzt, 12 Stunden stehen läßt, den gebildeten Niederschlag abzentrifugiert und in 1/8 bis 1/10 des ursprünglichen Volumens destilliertem Wasser aufnimmt, 2 Tage gegen entmineralisiertes Wasser dialysiert und anschließend lyophilisiert,

j) den Überstand der ersten Alkohol-Fällung mit dem 1,5 - 2,5-fachen, vorzugsweise 2-fachen Volumen Ethanol versetzt, die Lösung für 48 Stunden stehen läßt, den Niederschlag abzentrifugiert, in destilliertem Wasser löst, die Lösung 48 Stunden gegen entmineralisiertes Wasser dialysiert und anschließend lyophilisiert.

2. Verfahren zur Herstellung der Polysaccharide nach Anspruch 1, **dadurch gekennzeichnet,** daß man

a) sterilisierte Keimlinge, Blatt-, Blüten-, Stengel- und Wurzelteile pflanzlichen Ausgangsmaterials von Echinacea purpurea (Linné) und Echinacea angustifolia (De Vandolle) als Ausgangsmaterial verwendet,

b) sie zur Kallusbildung in ein agarhaltiges Nährmedium überführt,

c) aus dem gebildeten Kallus Primärzellen für eine Zellsuspension nach an sich bekannten Methoden gewinnt,

d) die Zellsuspensionskultur abfiltriert,

e) das Filtrat mit dem 2 - 4-fachen, vorzugsweise dreifachen, Volumen Ethanol versetzt, den gebildeten Niederschlag zunächst durch Abdekantieren, anschließend durch Abzentrifugieren abtrennt,

f) das Zentrifugat in Wasser löst, unlösliche Bestandteile abzentrifugiert, den Überstand mit dem 0,5 - 1,5-fachen, vorzugsweise 1-fachen Volumen Ethanol versetzt und für 48 Stunden stehen läßt,

g) den gebildeten Niederschlag abzentrifugiert, anschließend in destilliertem Wasser löst, die Lösung 48 Stunden gegen demineralisiertes Wasser dialysiert und anschließend lyophilisiert,

h) den Überstand der ersten Ethanol-Fällung mit dem 1,5 - 2,5-fachen, vorzugwsweise 2-fachen Volumen Ethanol versetzt, 12 Stunden stehen läßt, den gebildeten Niederschlag abzentrifugiert, anschließend in destilliertem Wasser löst, 48 Stunden gegen entmineralisiertes Wasser dialysiert und anschließend lyophilisiert.

3. Verfahren nach Anspruch 1 oder 2 zur Feinreinigung der Polysaccharide, **dadurch gekennzeichnet,** daß man die Polysaccharide aus der 1 : 1 Fällung mit Ethanol folgenden Reinigungsschritten unterwirft:

a) einer Anionenaustauschchromatographie in wässriger Lösung,

b) mit $H_2O$ als Elutionsmittel eine erste Polysaccharidfraktion isoliert und

c) weitere, saure Polysaccharide mittels eines NaCl-Gradienten von 0 bis 0,75 M eluiert.

4. Verfahren nach Anspruch 1 oder 2 , **dadurch gekennzeichnet,** daß man die neutralen Polysaccharide aus der 1 : 1 Fällung mittels folgender Reinigungsschritte isoliert:

a) die Polysaccharide zunächst einer Anionenaustauschchromatographie mit $H_2O$ als Elutionsmittel, unterwirft,

b) die vereinigten polysaccharidhaltigen Fraktionen einer weiteren Anionenaustauschchromatographie unterwirft, mit $H_2O$ eluiert und

c) die vereinigten Polysaccharidfraktionen durch Gelchromatographie unter Verwendung von $H_2O$ oder 0,2 M NaCl als Eluierungsmittel weiter reinigt.

5. Verfahren nach Anspruch 1 oder 2, **dadurch gekennzeichnet,** daß man die Polysaccharide aus der 1 : 4 Fällung durch folgende Reinigungsschritte isoliert:

a) zunächst durch Anionenaustauschchromatographie das neutrale Polysaccharid unter Verwendung von $H_2O$ als Eluierungsmittel abtrennt, anschließend b) unter Verwendung eines NaCl Konzentrationsgradienten von 0 bis 0,4 M das saure Polysaccharid eluiert, weiter

c) das neutrale Polysaccharid durch Gelfiltrationschromatographie unter Verwendung von 0,2 M NaCl oder $H_2O$ als Eluierungsmittel weiter reinigt, und

d) das saure Polysaccharid einer weiteren Anionenaustauschchromatographie unter Verwendung von zunächst $H_2O$ um zufällig vorhandene neutrale Polysaccharide abzutrennen, dann das saure Polysaccharid unter Verwendung eines NaCl-Gradienten von 0 bis 0,4 M eluiert, und

e) beide Polysaccharide einer weiteren Gelfiltrationschromatographie unter Verwendung von 0,2 M NaCl-Lösung als Elutionsmittel unterwirft.

6. Verfahren nach Anspruch 1 oder 2 , **dadurch gekennzeichnet,** daß man zur Ausfällung der Polysaccharide Aceton, Ammoniumsulfat, Cetyltrimethylammoniumbromid, $Ca_2Cl_2$ oder Fehling'sche Lösung verwendet.

7. Verfahren zur Herstellung der Polysaccharide nach Anspruch 1 bis 6, **dadurch gekennzeichnet,** daß man
a) sterillsierte Keimlinge, Blatt-, Blüten-, Stengel- und Wurzelteile pflanzlichen Ausgangsmaterials von Echinacea purpurea und Echinacea angustifolia als Ausgangsmaterial verwendet, diese zur Kallusbildung in ein agarhaltiges Nährmedium überführt, aus dem gebildeten Kallus Primärzellen für eine Zellsuspension nach an sich bekannten Methoden gewinnt, die Zellsuspensionskulturen nach 12 - 20, vorzugsweise 14 Tagen bei 22 - 28° C abfiltriert, die Zellrückstände verwirft,
b) Resttrübungen des Nährmediums durch Zellfragmente oder Eiweißausscheidungen durch Klärung im Durchlaufseparator, vorzugsweise in einem Kammer- oder Tellerseparator, entfernt und die abgetrennten Feststoffe verwirft,
c) kolloidal gelöste Bestandteile sowie hochmolekulare Verbindungen (Molekulargewichte > $10^6$ Dalton) durch Mikrofiltration entfernt, wobei vorzugsweise Polysulfonmembranen in Rohr- oder Kapillarmodulen mit einer Porengröße ≧ 0,1 µm oder Ultrafiltrationsmembranen mit Trenngrenzen > $10^5$ Dalton verwendet werden,
d) das Retentat bei Erreichen eines vorgegebenen Minimalvolumens mit demineralisiertem Wasser diafiltriert, Permeat und Diafiltrat vereinigt, das Retentat verwirft und die vereinigten Permeate durch Ultrafiltration aufkonzentriert (Konzentrationsfaktoren zwischen 10 und 500, je nach Ausgangsvolumen und Anlagengröße), anschließend bis zum Erreichen einer Leitfähigkeit von unter 100 µS/cm mit demineralisiertem Wasser diafiltriert und das Permeat verwirft,
e) das konzentrierte Retentat durch
e.1 Erhitzen auf Temperaturen von 90° C bis 130° C und
e.2 nach dem Abkühlen zur Abtrennung der Niederschläge filtriert oder zentrifugiert oder dadurch enteiweißt daß man
e.1 die Lösung mit Trichloressigsäure (TCA) bis zu einer Konzentration von 7,5 Gew.% versetzt,
e.2 bei 4° C mindestens 12 Stunden stehen läßt,
e.3 die Niederschläge abfiltriert oder zentrifugiert,
e.4 die geklärte Lösung bis zu einer Leitfähigkeit < 100 µS/cm gegen demineralisiertes Wasser dialysiert,
f) das konzentrierte Retentat am schwachen Anionenaustauscher zunächst mit Wasser als Eluierungsmittel zur Gewinnung der neutralen Polysaccharidfraktion (entsprechend Polysaccharidfraktion A) und anschließend mit einem Salzgradienten (Natriumacetat, NaCl, etc.) von 0 bis 4 molar zur Gewinnung der sauren Polysaccharidfraktionen (entsprechend Polysaccharidfraktion F) eluiert,
g) die Einzelfraktionen gegebenenfalls wie oben unter e) enteiweißt,
h) die $Einzelfraktionen_1$ wie unter d), einer Ultra- und Diafiltration unterwirft und
i) anschließend die Produkte durch Lyophilisation, Vakuumtrocknung oder Sprühtrocknung trocknet.

8. Verfahren nach Anspruch 7, **dadurch gekennzeichnet,** daß man die Enteiweißung gemäß Stufe e) bereits im Anschluß an die Stufe a) durchführt.

9. Verfahren nach Anspruch 7 oder 8, **dadurch gekennzeichnet,** daß man auf Stufe d) Membranen aus Polysulfon oder Celluloseacetat in Rohr-, Kapillar- oder Spiralwickelmodulen verwendet, die Trenngrenzen von 10 000 Dalton oder weniger aufweisen.

10. Verfahren nach Anspruch 7 bis 9, **dadurch gekennzeichnet,** daß man auf Stufe d) anstelle der Dialfiltration eine Entsalzung durch Dialyse oder Gelfiltration durchführt.

11. Verfahren zur Herstellung einer pharmazeutischen Zubereitung, **dadurch gekennzeichnet,** daß man die Polysaccharide, hergestellt nach einem der Ansprüche 1 bis 10 mit üblichen, pharmazeutisch verträglichen Träger- und Hilfsstoffen vermischt.

## Claims

**Claims for the following Contracting States : DE, NL, GB, CH, LI, FR, LU, BE, SE, IT**

1. Polysaccharides having immuno-stimulating activity, **characterized in that** they have an average molecular weight of 10,000 with a molecular weight range of from 5,000 to 15,000 of the general structure:

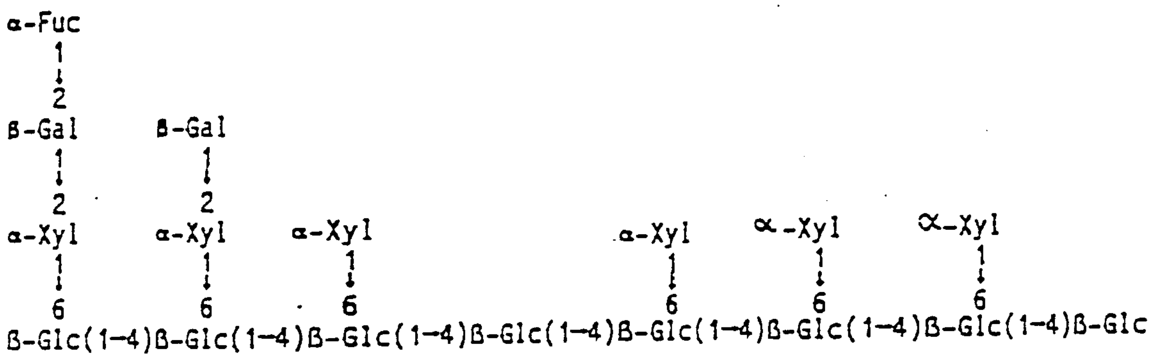

2. Polysaccharides having immuno-stimulating activity, **characterized in that** they have an average molecular weight of 25,000 with a molecular weight range of from 20,000 to 30,000 of the following general structure:

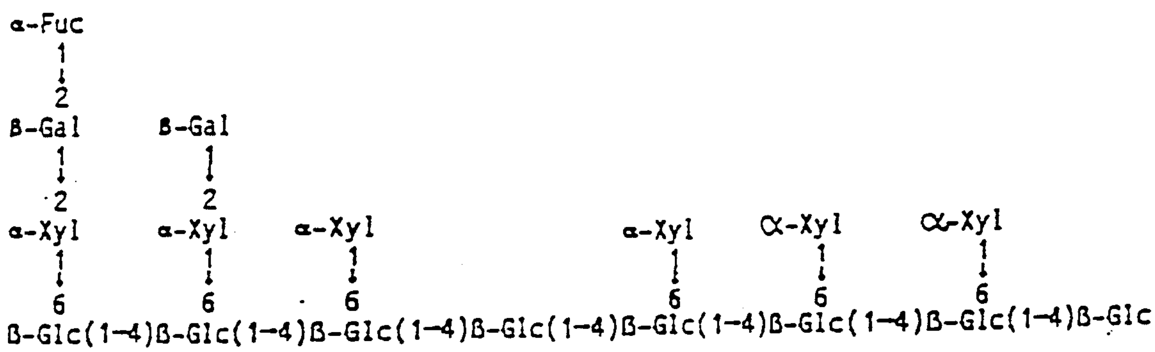

3. Polysaccharides having immuno-stimulating activity, **characterized in that** they have an average molecular weight of 110,000 as measured in 0.2 M phosphate buffer with a range of from 100,000 to 120,000, and that their general structure essentially consists of the following moieties:

a) an arabinogalactan moiety of the formula:

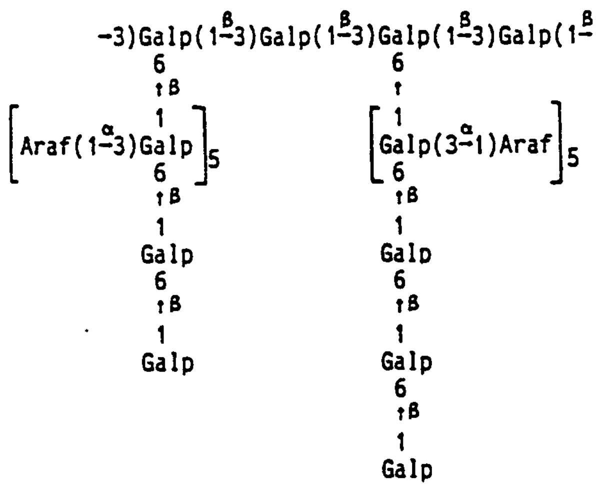

b) a rhamnogalacturonan moiety of the formula:

$-2)Rha(1-4)GalA(1\overset{\alpha}{-}4)GalA(1\overset{\alpha}{-}4)GalA(1\overset{\alpha}{-}2)Rha(1-4)GalA(1\overset{\alpha}{-}$

c) an arabinan moiety of the formula:

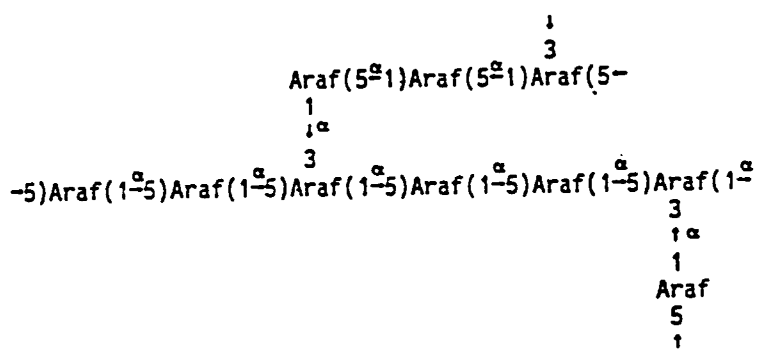

4. The polysaccharides according to claims 1 to 3, **characterized in that** they are isolated from plant cell cultures of Echinacea purpurea (Linné) Moench and Echinacea angustifolia (De Vandolle).

5. A process for preparing the polysaccharides of claims 1 to 4, **characterized in that** it comprises the following steps of:
   a) selecting sterilized seedlings, leaf, flower, stalk or root parts of vegetable origin from Echinacea purpurea (Linné) and Echinacea angustifolia (De Vandolle) for use as starting materials,
   b) transferring said starting materials into an agar-containing culture medium for callus formation,
   c) obtaining primary cells from said callus for preparing a cell suspension applying methods which are per se already known,
   d) filtering said cell suspension cultures after incubation for 12 to 20 days, preferrably for 14 days, at 22 - 28 °C, and lyophilizing the cellular residues for determination of weight,
   e) mixing the cell culture supernatant with 2-4 times, preferrably 3 times, its volume of ethanol,
   f) separating the resulting precipitate by centrifugation, subsequently dissolving it in distilled water,
   g) mixing it with 15 % trichloroacetic acid to a final concentration of 7.5 %, allowing it to stand and then centrifuging it,

h) mixing the supernatant obtained after the centrifugation with 3 - 6 times, preferrably 4 times, its volume of ethanol, centrifuging the resulting precipitate after having been allowed to stand for 12 hours, subsequently dissolving it in a 2 % sodium acetate solution, then stirring this solution for 8 hours and subsequently filtering it,

i) mixing said filtrate with 0.5-1.5 times, preferrably 1 time, its volume of ethanol, allowing it to stand for 12 hours, centrifuging the resulting precipitate and dissolving it in 1/8 to 1/10 of the original volume of distilled water, dialyzing it against demineralized water for 2 days and subsequently lyophilizing it,

j) mixing the supernatant from the first alcohol precipitation with 1.5 - 2.5 times, preferrably 2 times, its volume of ethanol, allowing the solution to stand for 48 hours, centrifuging the resulting precipitate, dissolving it in distilled water, dialyzing the solution against demineralized water for 48 hours and subsequently lyophilizing it.

6. A process for preparing the polysaccharides of claims 1 to 4, **characterized in that** it comprises the following steps of:

a) selecting sterilized seedlings, leaf, flower, stalk or root parts of vegetable origin from Echinacea purpurea (Linné) and Echinacea angustifolia (De Vandolle) for use as starting materials,

b) transferring said starting materials into an agar-containing culture medium for callus formation,

c) obtaining primary cells from said callus for preparing a cell suspension applying methods which are per se already known,

d) filtering said cell suspension cultures,

e) mixing the filtrate with 2-4 times, preferrably 3 times, its volume of ethanol, separating the resulting precipitate first by means of decantation and subsequently by means of centrifugation,

f) dissolving the centrifugate in water, centrifuging unsoluble constituents, mixing the supernatant with 0.5-1.5 times, preferrably 1 time, its volume of ethanol and allowing it to stand for 48 hours,

g) centrifuging the resulting precipitate, subsequently dissolving it in distilled water, dialyzing the solution against demineralized water for 48 hours and subsequently lyophilizing it,

h) mixing the supernatant of the first ethanol precipitation with 1.5 to 2.5, preferrably 2 times, its volume of ethanol, allowing it to stand for 12 hours, centrifuging the resulting precipitate, subsequently dissolving it in distilled water, dialyzing it against demineralized water for 48 hours and subsequently lyophilizing it.

7. A process according to claims 5 or 6 for the fine purification of the polysaccharides, **characterized in that** the polysaccharides from the 1 : 1 precipitation with ethanol are subjected to the following purification steps:

a) anion exchange chromatography in an aqueous solution,

b) isolation of a first polysaccharide fraction using $H_2O$ as the eluant,

c) elution of additional, acidic polysaccharides using an NaCl gradient of 0 to 0.75 M.

8. A process according to claims 5 or 6, **characterized in that** the neutral polysaccharides from the 1 : 1 precipitation are isolated by means of the following purification steps:

a) subjecting the polysaccharides first to an anion exchange chromatography using $H_2O$ as the eluant,

b) subjecting the combined polysaccharide-containing fractions to a further anion exchange chromatography, then to elution using $H_2O$ as the eluant, and

c) further purifying the combined polysaccharide fractions by means of gel chromatography using either $H_2O$ or 0.2 M NaCl as the eluant.

9. A process according to claims 5 or 6, **characterized in that** the polysaccharides from the 1 : 4 precipitation are isolated by means of the following purification steps:

a) first separating the neutral polysaccharide by means of anion exchange chromatography using $H_2O$ as the eluant, subsequently

b) eluting the acidic polysaccharide using an NaCl concentration gradient of 0 to 0.4 M, and further

c) subjecting the neutral polysaccharide to a further purification by means of gel filtration chromatography using 0.2 M NaCl or $H_2O$ as the eluant, and

d) subjecting the acidic polysaccharide to a further anion exchange chromatography first using $H_2O$ as an eluant so as to separate any residual neutral polysaccharides, and then eluting the acidic polysaccharide using an NaCl gradient of 0 to 0.4 M, and

e) subjecting both polysaccharides to a further gel filtration chromatography using 0.2 M NaCl solution as the eluant.

**10.** A process according to claims 5 or 6, **characterized in that** acetone, ammonium sulfate, cetyl trimethyl ammonium bromide, calcium chloride, or Fehling's solution is used to precipitate the polysaccharides.

**11.** A process for preparing the polysaccharides of claims 1 to 4, **characterized by** the following steps of:
a) using sterilized seedlings, leaf, flower, stark and root parts of vegetable origin from Echinacea purpurea and Echinacea angustifolia as starting material, transferring said starting materials into an agar-containing culture medium for callus formation, obtaining primary cells from said callus for a cell suspension by applying methods which are per se known, filtering said cell suspension cultures after 12 - 20, preferrably after 14 days, at 22 - 28 °C, discarding the cellular residues,
b) removing residual turbidity of the culture medium due to cell fragments or due to precipitated protein by means of clarification in a flow-through separator, preferrably in a chamber or plate separator, discarding the separated solid particles,
c) removing colloidally dissolved components as well as high-molecular-weight compounds (molecular weights > $10^6$ Dalton) by means of microfiltration, preferrably using membranes made from polysulfone in tubular or capillary modules having a pore size of ≧ 0.1 µm or ultrafiltration membranes with separation limits of > $10^5$ Dalton,
d) diafiltering the retentate with demineralized water when a predetermined minimum volume is reached, combining the permeate fraction with the diafiltrate, discarding the retentate and concentrating the combined permeates by means of ultrafiltration (concentration factors between 10 and 500 depending on the starting volume and size of the device), subsequently diafiltering them with demineralized water until a conductivity of below 100 µS/cm is reached, and discarding the permeate,
e) then filtering or centrifuging the concentrated retentate
e. 1 by heating it to temperatures of from 90 °C to 130 °C and
e. 2 after the cooling for separating the precipitates
or deproteinizing it by means of
e. 1 mixing the solution with trichloroacetic acid (TCA) up to a concentration of 7.5 wt. %,
e. 2 allowing it to stand at 4 °C for at least 12 hours,
e. 3 filtering or centrifuging the precipitates,
e. 4 dialyzing the clarified solution against demineralized water until a conductivity of < 100 µS/cm is reached,
f) eluting the concentrated retentate by a relatively weak anion exchanger first using water as the eluant to obtain the neutral polysaccharide fraction (corresponding to polysaccaride fraction A) and subsequently by using a salt gradient (sodium acetate, NaCl, etc.) with 0 to 4 molar for obtaining the acidic polysaccharide fractions (corresponding to polysaccharide fraction F),
g) optionally deproteinizing the individual fraction as described above in step e),
h) subjecting the individual fractions to an ultrafiltration and to a diafiltration, as described in step d),
i) subsequently drying the products by lyophilization, vacuum drying or spray drying.

**12.** A process according to claim 11, **characterized in that** the deproteinization of step e) is already carried out subsequent to step a).

**13.** A process according to claims 11 to 12, **characterized in that** step d) is carried out using membranes made from polysulfone or cellulose acetate in tubular, capillary or spiral coil modules, said membranes having separation limits of 10,000 Dalton or less.

**14.** A process according to claims 11 to 13, **characterized in that** in step d) the diafiltration is replaced by desalting by means of dialysis or gel filtration.

**15.** A pharmaceutical preparation, **characterized in that** it contains the polysaccharides of claims 1 to 4 along with the common pharmaceutically acceptable carrier and auxiliary substances.

**Claims for the following Contracting States : AT, ES, GR**

1. A process for preparing polysaccharide from plant cell structures of Echinacea purpurea (Linné) and Echinacea angustifolia (De Vandolle), having the following general structures (I), (II) and (III):

(I): an average molecular weight of 10,000 with a molecular weight range of from 5,000 - 15,000 and the following general structure:

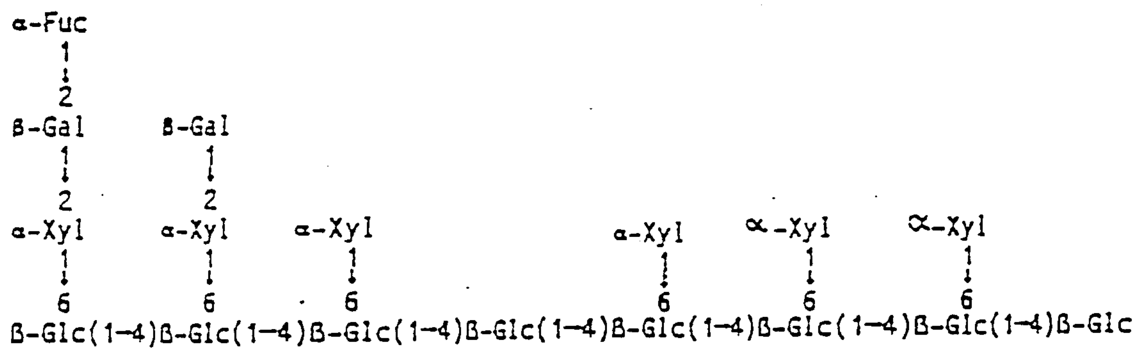

(II): an average molecular weight of 25,000 with a molecular weight range of from 20,000 to 30,000 and the following general structure:

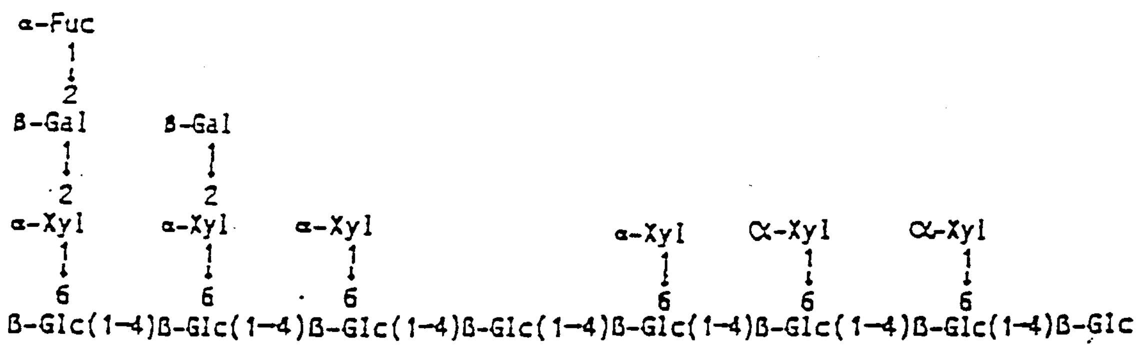

(III): an average molecular weight of 110,000 as measured in 0.2 M phosphate buffer with a range of from 100,000 to 120,000, and wit a general structure consisting essentially of the following moieties:

a) an arabinogalactan moiety of the formula:

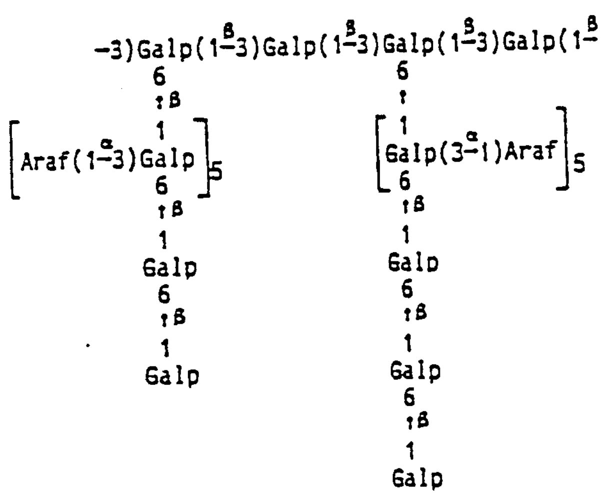

b) a rhamnogalacturonan moiety of the formula:

$$-2)Rha(1-4)GalA(1\overset{\alpha}{-}4)GalA(1\overset{\alpha}{-}4)GalA(1\overset{\alpha}{-}2)Rha(1-4)GalA(1\overset{\alpha}{-}$$

c) an arabinan moiety of the formula:

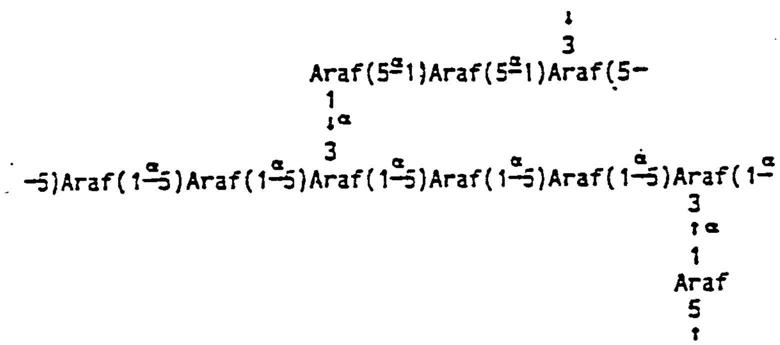

**characterized in that** it comprises the following steps of:

a) selecting sterilized seedlings, leaf, flower, stalk or root parts of vegetable origin from Echinacea purpurea (Linné) and Echinacea angustifolia (De Vandolle) for use as starting materials,

b) transferring said starting materials into an agar-containing culture medium for callus formation,

c) obtaining primary cells from said callus for preparing a cell suspension applying methods which are per se already known,

d) filtering said cell suspension cultures after incubation for 12 to 20 days, preferably for 14 days, at 22 - 28 °C, and lyophilizing the cellular residues for determination of weight,

e) mixing the cell culture supernatant with 2-4 times, preferably 3 times, its volume of ethanol,

f) separating the resulting precipitate by centrifugation, subsequently dissolving it in distilled water,

g) mixing it with 15 % trichloroacetic acid to a final concentration of 7.5 %, allowing it to stand and then centrifuging it,

h) mixing the supernatant obtained after the centrifugation with 3 - 6 times, preferably 4 times, its volume of ethanol, centrifuging the resulting precipitate after having been allowed to stand for 12 hours, subsequently dissolving it in a 2 % sodium acetate solution, then stirring this solution for 8 hours and subsequently filtering it,

i) mixing said filtrate with 0.5-1.5 times, preferably 1 time, its volume of ethanol, allowing it to stand for 12 hours, centrifuging the resulting precipitate and dissolving it in 1/8 to 1/10 of the original volume of distilled water, dialyzing it against demineralized water for 2 days and subsequently lyophilizing it,

j) mixing the supernatant from the first alcohol precipitation with 1.5 - 2.5 times, preferably 2 times, its volume of ethanol, allowing the solution to stand for 48 hours, centrifuging the resulting precipitate, dissolving it in distilled water, dialyzing the solution against demineralized water for 48 hours and subsequently lyophilizing it.

**2.** A process for preparing the polysaccharides according to claim 1, **characterized in that** it comprises the following steps of:

a) selecting sterilized seedlings, leaf, flower, stalk or root parts of vegetable origin from Echinacea purpurea (Linné) and Echinacea angustifolia (De Vandolle) for use as starting materials,

b) transferring said starting materials into an agar-containing culture medium for callus formation,

c) obtaining primary cells from said callus for preparing a cell suspension applying methods which are per se already known,

d) filtering said cell suspension cultures,

e) mixing the filtrate with 2-4 times, preferably 3 times, its volume of ethanol, separating the resulting precipitate first by means of decantation and subsequently by means of centrifugation,

f) dissolving the centrifugate in water, centrifuging unsoluble constituents, mixing the supernatant with 0.5-1.5 times, preferably 1 time, its volume of ethanol and allowing it to stand for 48 hours,

g) centrifuging the resulting precipitate, subsequently dissolving it in distilled water, dialyzing the solution against demineralized water for 48 hours and subsequently lyophilizing it,

h) mixing the supernatant of the first ethanol precipitation with 1.5 to 2.5, preferably 2 times, its volume of ethanol, allowing it to stand for 12 hours, centrifuging the resulting precipitate, subsequently dissolving it in distilled water, dialyzing it against demineralized water for 48 hours and subsequently lyophilizing it.

**3.** A process according to claims 1 or 2 for the fine purification of the polysaccharides, **characterized in that** the polysaccharides from the 1 : 1 precipitation with ethanol are subjected to the following purification steps:

a) anion exchange chromatography in an aqueous solution,

b) isolation of a first polysaccharide fraction using $H_2O$ as the eluant,

c) elution of additional, acidic polysaccharides using an NaCl gradient of 0 to 0.75 M.

**4.** A process according to claims 1 or 2, **characterized in that** the neutral polysaccharides from the 1 : 1 precipitation are isolated by means of the following purification steps:

a) subjecting the polysaccharides first to an anion exchange chromatography using $H_2O$ as the eluant,

b) subjecting the combined polysaccharide-containing fractions to a further anion exchange chromatography, then to elution using $H_2O$ as the eluant, and

c) further purifying the combined polysaccharide fractions by means of gel chromatography using either $H_2O$ or 0.2 M NaCl as the eluant.

**5.** A process according to claims 1 or 2, **characterized in that** the polysaccharides from the 1 : 4 precipitation are isolated by means of the following purification steps:

a) first separating the neutral polysaccharide by means of anion exchange chromatography using $H_2O$ as the eluant, subsequently

b) eluting the acidic polysaccharide using an NaCl concentration gradient of 0 to 0.4 M, and further

c) subjecting the neutral polysaccharide to a further purification by means of gel filtration chromatography using 0.2 M NaCl or $H_2O$ as the eluant, and

d) subjecting the acidic polysaccharide to a further anion exchange chromatography first using $H_2O$ as an eluant so as to separate any residual neutral polysaccharides, and then eluting the acidic polysaccharide using an NaCl gradient of 0 to 0.4 M, and

e) subjecting both polysaccharides to a further gel filtration chromatography using 0.2 M NaCl solution as the eluant.

**6.** A process according to claims 1 or 2, **characterized in that** acetone, ammonium sulfate, cetyl trimethyl ammonium bromide, calcium chloride, or Fehling's solution is used to precipitate the polysaccharides.

**7.** A process for preparing the polysaccharides of claims 1 to 6, **characterized by** the following steps of:

a) using sterilized seedlings, leaf, flower, stark and root parts of vegetable origin from Echinacea purpurea and Echinacea angustifolia as starting material, transferring said starting materials into an agar-containing culture medium for callus formation, obtaining primary cells from said callus for a cell suspension by applying methods which are per se known, filtering said cell suspension cultures after 12 - 20, preferably after 14 days, at 22 - 28 °C, discarding the cellular residues,

b) removing residual turbidity of the culture medium due to cell fragments or due to precipitated protein by means of clarification in a flow-through separator, preferably in a chamber or plate separator, discarding the separated solid particles,

c) removing colloidally dissolved components as well as high-molecular-weight compounds (molecular weights $10^6$ Dalton) by means of microfiltration, preferably using membranes made from polysulfone in tubular or capillary modules having a pore size of = 0.1 μm or ultrafiltration membranes with separation limits of $10^5$ Dalton,

d) diafiltering the retentate with demineralized water when a predetermined minimum volume is reached, combining the permeate fraction with the diafiltrate, discarding the retentate and concentrating the combined permeates by means of ultrafiltration (concentration factors between 10 and

500 depending on the starting volume and size of the device), subsequently diafiltering them with demineralized water until a conductivity of below 100 µS/cm is reached, and discarding the permeate,

e) then filtering or centrifuging the concentrated retentate

e. 1 by heating it to temperatures of from 90 ° C to 130 ° C and

e. 2 after the cooling for separating the precipitates

or deproteinizing it by means of

e. 1 mixing the solution with trichloroacetic acid (TCA) up to a concentration of 7.5 wt. %,

e. 2 allowing it to stand at 4 ° C for at least 12 hours,

e. 3 filtering or centrifuging the precipitates,

e. 4 dialyzing the clarified solution against demineralized water until a conductivity of < 100 µS/cm is reached,

f) eluting the concentrated retentate by a relatively weak anion exchanger first using water as the eluant to obtain the neutral polysaccharide fraction (corresponding to polysaccaride fraction A) and subsequently by using a salt gradient (sodium acetate, NaCl, etc.) with 0 to 4 molar for obtaining the acidic polysaccharide fractions (corresponding to polysaccharide fraction F),

g) optionally deproteinizing the individual fractions as described above in step e),

h) subjecting the individual fractions to an ultrafiltration and to a diafiltration, as described in step d),

i) subsequently drying the products by lyophilization, vacuum drying or spray drying.

**8.** A process according to claim 7, **characterized in that** the deproteinization of step e) is already carried out subsequent to step a).

**9.** A process according to claims 7 or 8, **characterized in that** step d) is carried out using membranes made from polysulfone or cellulose acetate in tubular, capillary or spiral coil modules, said membranes having separation limits of 10,000 Dalton or less.

**10.** A process according to claims 7 to 9, **characterized in that** in step d) the diafiltration is replaced by desalting by means of dialysis or gel filtration.

**11.** A process for preparing a pharmaceutical preparation, **characterized in that** the polysaccharides prepared according to any of claims 1 to 10 are mixed with common pharmaceutically acceptable carrier and auxiliary substances.

## Revendications

**Revendications pour les Etats contractants suivants : DE, NL, GB, CH, LI, FR, LU, BE, SE, IT**

**1.** Polysaccharides à effet immunostimulant caractérisés en ce qu'ils possèdent un poids moléculaire moyen de 10.000 dans une gamme de 5.000 à 15.000 et présentent la structure de base suivante:

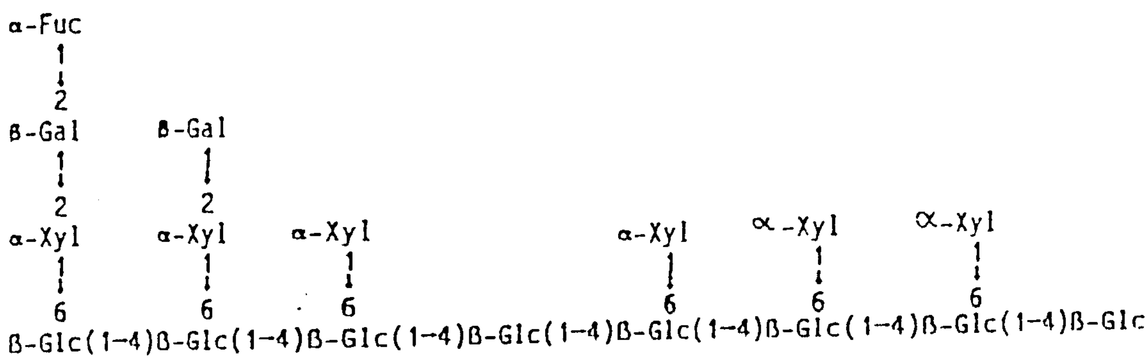

2. Polysaccharides à effet immunostimulant caractérisés en ce qu'ils possèdent un poids moléculaire moyen de 25.000 dans une gamme de 20.000 à 30.000 et présentent la structure de base suivante:

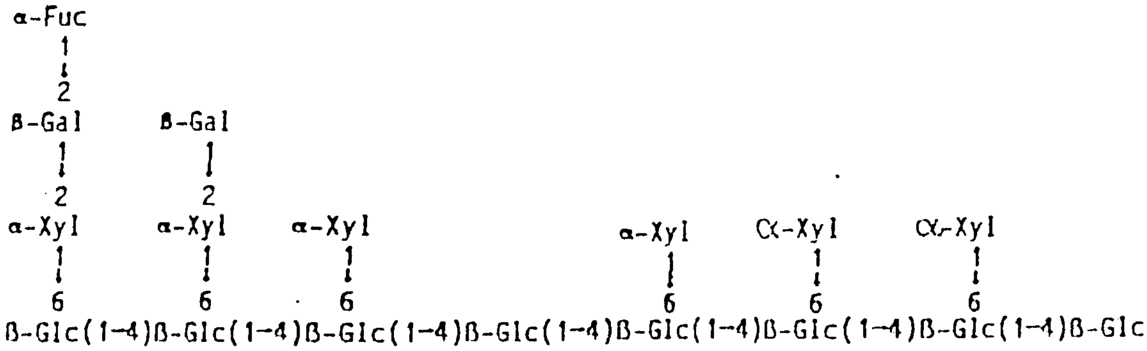

3. Polysaccharides à effet immunostimulant caractérisés en ce que leur poids moléculaire moyen dans un tampon phosphate 0,2M s'élève à 110.000 dans une gamme de 100.000 à 120.000 et en ce que leur structure de base se compose essentiellement des parties suivantes:

a) un radical arabinogalacto de formule:

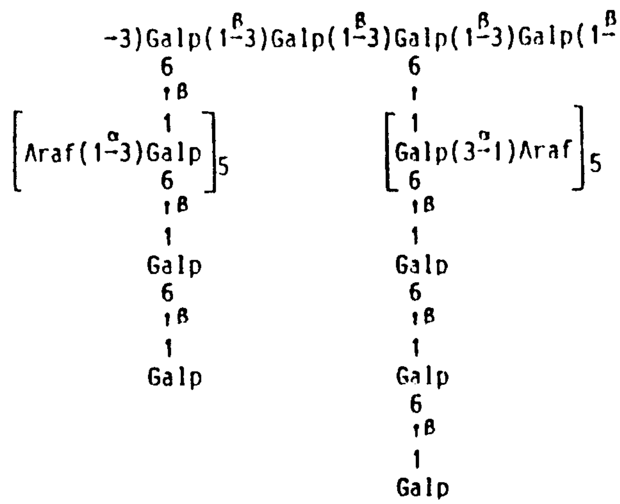

b) un radical rhamnogalacturonane de formule:

$-2)Rha(1-4)GalA(1\overset{\alpha}{-}4)GalA(1\overset{\alpha}{-}4)GalA(1\overset{\alpha}{-}2)Rha(1-4)GalA(1\overset{\alpha}{-}$

c) un radical arabinane de formule:

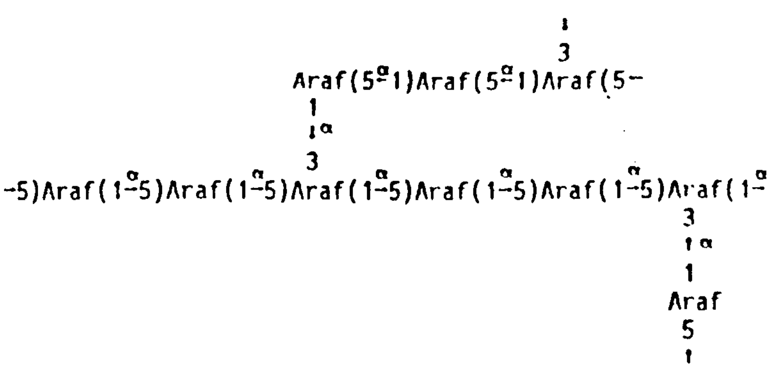

4. Polysaccharides, selon une des revendications 1 à 3, caractérisés en ce qu'ils ont été isolés à partir de cultures cellulaires végétales d'Echinacea purpurea (Linné) Moench et Echinacea angustifolia (De Vandolle).

5. Procédé de préparation de polysaccharides, selon une des revendications 1 à 4, caractérisé en ce que:
(a) des plantules, particules de feuilles, de fleurs, de tiges et de racines stérilisées de substances végétales de départ d'Echinacea purpurea (Linné) et Echinacea angustifolia (De Vandolle) sont utilisées comme substances de départ,
(b) celles-ci sont amenées dans un bouillon de culture contenant de l'agar pour la formation de cal,
(c) des cellules primaires pour une suspension cellulaire sont extraites selon des méthodes connues à partir du cal formé,
(d) les cultures de suspensions cellulaires sont séparées par filtration après 12 à 20, de préférence 14 jours à 22 - 28° C, les résidus cellulaires sont lyophilisés pour la détermination du poids,
(e) le surnageant de la culture cellulaire est mélangé avec deux à quatre fois, de préférence avec trois fois son volume d'éthanol,
(f) le dépôt formé est séparé par centrifugation et ensuite recueilli dans de l'eau distillée,
(g) le tout est mélangé avec de l'acide trichloroacétique à 15 % jusqu'à une concentration finale de 7,5 %, est amené à reposer et est ensuite centrifugé,
(h) le surnageant obtenu après centrifugation est mélangé avec trois à six fois, de préférence quatre fois son volume d'éthanol, le dépôt formé est centrifugé après 12 heures d'attente et est ensuite recueilli dans une solution d'acétate de sodium à 2 %, cette solution est agitée pendant 8 heures et ensuite filtrée,
(i) le produit filtré est mélangé avec 0,5 à 1,5 fois, de préférence 1 fois son volume d'éthanol, est amené à reposer pendant 12 heures, le dépôt formé est séparé par centrifugation et recueilli dans 1/8 à 1/10 du volume d'origine d'eau distillée, est dialysé pendant 2 jours à l'eau déminéralisée et ensuite lyophilisé.
(j) le surnageant de la première précipitation dans l'alcool est mélangé avec 1,5 à 2,5 fois, de préférence 2 fois son volume d'éthanol, la solution est amenée à reposer pendant 48 heures, le dépôt est séparé par centrifugation et dissous dans de l'eau distillée, la solution est dialysée pendant 48 heures à l'eau déminéralisée et est ensuite lyophilisée.

6. Procédé de préparation de polysaccharides selon une des revendications 1 à 4 caractérisé en ce que
(a) des plantules, particules de feuilles, de fleurs, de tiges et de racines stérilisées de substances de départ végétales d'Echinacea purpurea (Linné) et Echinacea angustifolia (De Vandolle) sont utilisées comme substances de départ,
(b) celles-ci sont amenées dans un bouillon de culture contenant de l'agar pour la formation de cal,
(c) des cellules primaires pour une suspension cellulaire sont obtenues selon des méthodes connues à partir du cal formé,
(d) la culture de suspensions cellulaires est séparée par filtration,
(e) le produit filtré est mélangé avec 2 à 4 fois de préférence 3 fois son volume d'éthanol, le dépôt formé est d'abord séparé par décantation et ensuite par centrifugation,
(f) le produit centrifugé est dissous dans l'eau, les particules insolubles sont séparées par centrifugation, le surnageant est mélangé dans 0,5 à 1,5 fois, de préférence 1 fois son volume d'éthanol et amené à reposer pendant 48 heures,
(g) le dépôt formé est séparé par centrifugation, dissous ensuite dans de l'eau distillée, la solution est dialysée pendant 48 heures à l'eau déminéralisée et ensuite lyophilisée.
(h) le surnageant de la première précipitation dans l'éthanol est mélangé avec 1,5 à 2,5 fois, de préférence 2 fois son volume d'éthanol, la solution est amenée à reposer pendant 12 heures, le précipité formé est séparé par centrifugation et dissous dans de l'eau distillée, la solution est dialysée pendant 48 heures à l'eau déminéralisée et ensuite lyophilisée.

7. Procédé, selon la revendication 5 ou 6, de purification des polysaccharides caractérisé en ce que les polysaccharides provenant de la précipitation 1 : 1 à l'éthanol sont soumis aux étapes de purification suivantes:
a) une chromatographie par échange d'anions dans une solution aqueuse,
b) une première fraction de polyssacharides est isolée avec de l'$H_2O$ comme éluant et
c) d'autres polysaccharides, acides, sont élués à l'aide d'un gradient de NaCl de 0 à 0,75 M.

**8.** Procédé, selon la revendication 5 ou 6, caractérisé en ce que les polysaccharides neutres de la précipitation 1 : 1 sont isolés par les étapes de purification suivantes:

a) les polysaccharides sont d'abord soumis à une chromatographie par échange d'anions avec de l'$H_2O$ comme éluant,

b) les fractions purifiées contenant des polysaccharides sont soumises à une autre chromatographie par échange d'anions, éluées avec de l'$H_2O$ et

c) les fractions purifiées de polysaccharides sont encore purifiées par chromatographie sur gel en utilisant de l'$H_2O$ ou du NaCl 0,2M comme éluant.

**9.** Procédé selon la revendication 5 ou 6 caractérisé en ce que les polysaccharides de la précipitation 1 : 4 sont isolés par les étapes de purification suivantes:

a) le polysaccharide neutre est d'abord séparé par chromatographie par échange d'anions en utilisant de l'$H_2O$ comme éluant, ensuite

b) le polysaccharide acide est élué en utilisant un gradient de concentration de NaCl de 0 à 0,4 M, ensuite

c) le polysaccharide neutre est encore purifié par chromatographie par filtration sur gel en utilisant du NaCl 0,2M ou de l'$H_2O$ comme éluant et

d) le polysaccharide acide est d'abord soumis à une autre chromatographie par échange d'anions en utilisant d'abord l'$H_2O$ pour séparer le polysaccharide neutre accidentellement présent et il est ensuite élué en utilisant un gradient de NaCl de 0 à 0,4 M, et

e) les deux polysaccharides sont soumis à une autre chromatographie par filtration sur gel en utilisant une solution de NaCl 0,2M comme éluant.

**10.** Procédé, selon la revendication 5 ou 6, caractérisé en ce que de l'acétone, du sulfate d'ammonium, du bromure de cétyltriméthylammonium, du $CaCl_2$ ou une solution de Fehling sont utilisés pour la précipitation des polysaccharides.

**11.** Procédé de préparation de polysaccharides, selon une des revendications 1 à 4, caractérisé en ce que

(a) des plantules, particules de feuilles, de fleurs, de tiges et de racines stérilisées de substances végétales de départ d'Echinacea purpurea et Echinacea angustifolia sont utilisées comme substances de départ; celles-ci sont amenées dans un bouillon de culture contenant de l'agar pour la formation de cal, des cellules primaires pour une suspension cellulaire sont extraites selon des méthodes connues à partir du cal formé; les cultures de suspensions cellulaires sont séparées par filtration après 12 à 20, de préférence 14 jours à 22 - 28 ° C, les résidus cellulaires sont rejetés,

(b) les troubles résiduels du bouillon de culture causés par des fragments cellulaires ou des dépôts protéinés sont éliminés par clarification dans un séparateur continu, de préférence dans un séparateur à chambre ou à disque et les particules séparées sont éliminées,

(c) les éléments dissous colloïdalement ainsi que les composés de poids moléculaire élevé (poids moléculaire > $10^6$ dalton) sont éliminés par microfiltration, des membranes de polysulfones dans des modules tubulaires ou capillaires avec une dimension des pores ≧ 0,1 µm ou des membranes d'ultrafiltration avec des limites de séparation > $10^5$ dalton étant utilisées,

d) une fois un volume minimal prédéterminé atteint, le rétentat est diafiltré avec de l'eau déminéralisée, le perméat et le diafiltrat sont purifiés, le rétentat est écarté et les perméats purifiés sont concentrés par ultrafiltration (facteurs de concentration entre 10 et 500, en fonction des volumes de départ et de l'importance de l'installation), le tout est ensuite diafiltré avec de l'eau déminéralisée jusqu'à une conductibilité inférieure à 100 µS/cm et le perméat est éliminé,

e) le rétentat concentré est,

e.1. en chauffant à des températures de 90 ° C à 130 ° C et

e.2. après refroidissement pour la séparation des dépôts, filtré ou centrifugé ou déprotéiné en

e.1. mélangeant la solution avec de l'acide trichloroacétique (TAC) jusqu'à une concentration de 7,5 % en poids,

e.2. la laissant reposer à 4 ° C pendant 12 heures au moins,

e.3. séparant par filtration ou en centrifugeant les dépôts,

e.4. en dialysant la solution clarifiée jusqu'à une conductibilité <100 µS/cm à l'eau déminéralisée,

f) le rétentat concentré est d'abord élué sur un échangeur d'anions faible avec de l'eau comme éluant pour extraire la fraction de polysaccharide neutre (correspondant à la fraction de polysaccharide A) et ensuite avec un gradient de sel (acétate de sodium, NaCl, etc.) de 0 à 4 molaire pour l'extraction de la fraction de polysaccharide acide (correspondant à la fraction de polysaccharide F),

g) les fractions individuelles sont éventuellement déprotéinées comme ci-dessus au point e),
h) comme au point d), les fractions individuelles subissent une ultrafiltration et une diafiltration et
i) les produits sont ensuite déshydratés par lyophilisation, séchage sous vide ou séchage par vaporisation.

**12.** Procédé, selon la revendication 11, caractérisé en ce que la déprotéination selon l'étape e) est déjà effectuée après l'étape a).

**13.** Procédé, selon la revendication 11 ou 12, caractérisé en ce que des membranes de polysulfone ou d'acétate de cellulose dans des modules tubulaires, capillaires ou spiralés qui présentent des limites de séparation de 10.000 dalton ou moins sont utilisées à l'étape d).

**14.** Procédé, selon une des revendications 11 à 13, caractérisé en ce que la diafiltration de l'étape d) est remplacée par une déminéralisation par dialyse ou filtration sur gel.

**15.** Préparation pharmaceutique caractérisée en ce qu'elle contient des polysaccharides, en plus des charges et adjuvants classiques compatibles pharmacologiquement, selon une des revendications 1 à 4.

**Revendications pour les Etats contractants suivants : AT, ES, GR**

**1.** Procédé de préparation de polysaccharides à partir de cultures cellulaires végétales d'Echinacea purpurea (Linné) Moench et Echinacea angustifolia (De Vandolle) qui présentent les structures de base suivante (I), (II) et (III):
(I): un poids moléculaire moyen de 10.000 dans une gamme de 5.000 à 15.000 et la structure de base suivante:

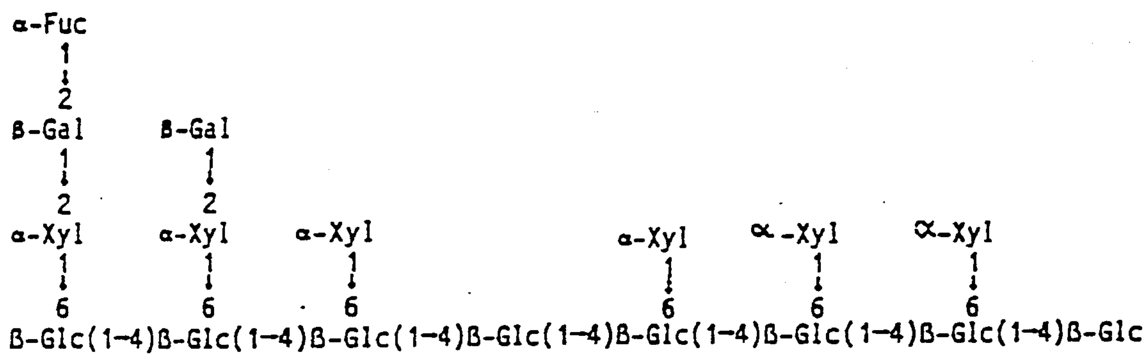

(II): un poids moléculaire moyen de 25.000 dans une gamme de 20.000 à 30.000 et la structure de base suivante:

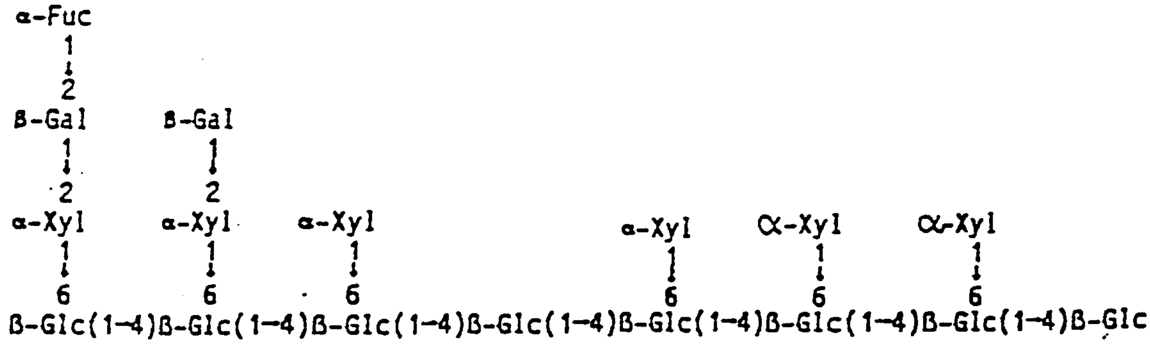

(III): un poids moléculaire moyen dans un tampon phosphate 0,2M de 110.000 dans une gamme de 100.000 à 120.000 et une structure de base qui se compose essentiellement des parties suivantes:

a) un radical arabinogalacto de formule:

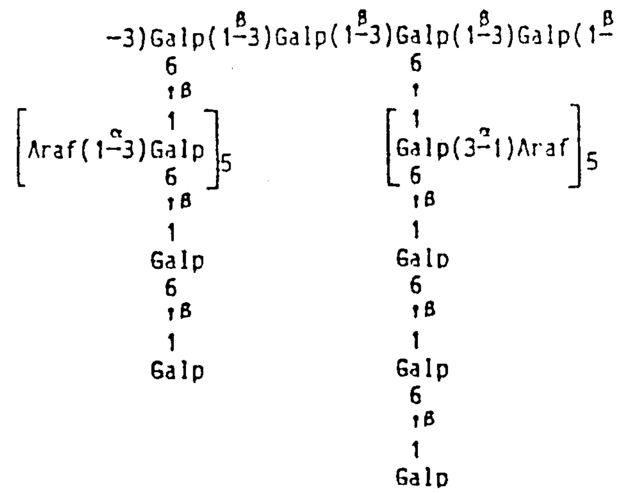

b) un radical rhamnogalacturonane de formule:

$$-2)Rha(1-4)GalA(1\overset{\alpha}{-}4)GalA(1\overset{\alpha}{-}4)GalA(1\overset{\alpha}{-}2)Rha(1-4)GalA(1\overset{\alpha}{-}$$

c) un radical arabinane de formule:

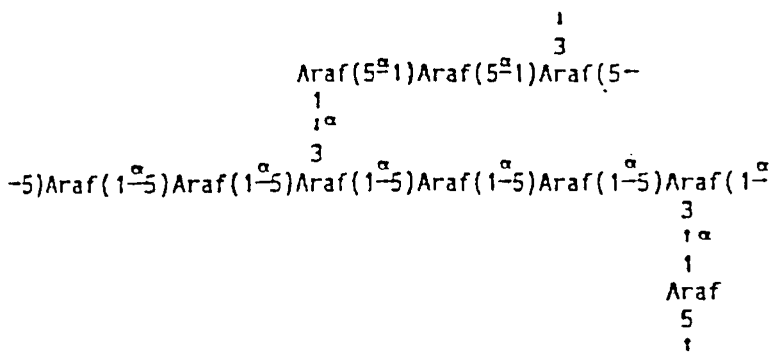

caractérisé en ce que

(a) des plantules, particules de feuilles, de fleurs, de tiges et de racines stérilisées de substances végétales de départ d'Echinacea purpurea (Linné) et Echinacea angustifolia (De Vandolle) sont utilisées comme substances de départ,

(b) celles-ci sont amenées dans un bouillon de culture contenant de l'agar pour la formation de cal,

(c) des cellules primaires pour une suspension cellulaire sont extraites selon des méthodes connues à partir du cal formé,

(d) les cultures de suspensions cellulaires sont séparées par filtration après 12 à 20, de préférence 14 jours à 22 - 28 ° C, les résidus cellulaires sont lyophilisés pour la détermination du poids,

(e) le surnageant de la culture cellulaire est mélangé avec deux à quatre fois son volume d'éthanol, de préférence trois fois son volume,

(f) le dépôt formé est séparé par centrifugation et ensuite recueilli dans de l'eau distillée,

(g) le tout est mélangé avec de l'acide trichloroacétique à 15 % jusqu'à une concentration finale de 7,5 %, est amené à reposer et est ensuite centrifugé,

(h) le surnageant obtenu après centrifugation est mélangé dans trois à six fois, de préférence quatre fois son volume d'éthanol, le dépôt formé est centrifugé après 12 heures d'attente, est ensuite recueilli dans une solution d'acétate de sodium à 2 %, cette solution est agitée pendant 8 heures et ensuite filtrée,

(i) le produit filtré est mélangé avec 0,5 à 1,5 fois, de préférence 1 fois le volume d'éthanol, est laissé à reposer pendant 12 heures, le dépôt formé est séparé par centrifugation, recueilli dans 1/8 à 1/10 du volume d'origine d'eau distillée, est dialysé pendant 2 jours à l'eau déminéralisée et ensuite lyophilisé.

(j) le surnageant de la première précipitation dans l'alcool est mélangé avec 1,5 à 2,5 fois, de préférence 2 fois son volume d'éthanol, la solution est amenée à reposer pendant 48 heures, le dépôt est séparé par centrifugation et dissous dans de l'eau distillée, la solution est dialysée pendant 48 heures à l'eau déminéralisée et ensuite lyophilisée.

**2.** Procédé de préparation de polysaccharides, selon la revendication 1, caractérisé en ce que

(a) des plantules, particules de feuilles, de fleurs, de tiges et de racines stérilisées de substances de départ végétales d'Echinacea purpurea (Linné) et Echinacea angustifolia (De Vandolle) sont utilisées comme substances de départ,

(b) celles-ci sont amenées dans un bouillon de culture contenant de l'agar pour la formation de cal,

(c) des cellules primaires pour une suspension cellulaire sont obtenues selon des méthodes connues à partir du cal formé,

(d) la culture de suspensions cellulaires est séparée par filtration,

(e) le produit filtré est mélangé avec 2 à 4 fois de préférence 3 fois son volume d'éthanol, le dépôt formé est d'abord séparé par décantation et ensuite par centrifugation,

(f) le produit centrifugé est dissous dans l'eau, les particules insolubles sont séparées par centrifugation, le surnageant est mélangé dans 0,5 à 1,5 fois, de préférence 1 fois son volume d'éthanol et amené à reposer pendant 48 heures,

(g) le dépôt formé est séparé par centrifugation, dissous ensuite dans de l'eau distillée, la solution est dialysée pendant 48 heures à l'eau déminéralisée et ensuite lyophilisée.

(h) le surnageant de la première précipitation dans l'éthanol est mélangé avec 1,5 à 2,5 fois, de préférence 2 fois son volume d'éthanol, la solution est amenée à reposer pendant 12 heures, le précipité formé est séparé par centrifugation et dissous dans de l'eau distillée, la solution est dialysée pendant 48 heures à l'eau déminéralisée et est ensuite lyophilisée.

**3.** Procédé, selon la revendication 1 ou 2, de purification des polysaccharides caractérisé en ce que les polysaccharides provenant de la précipitation 1 : 1 à l'éthanol sont soumis aux étapes de purification suivantes:

a) ils subissent une chromatographie par échange d'anions dans une solution aqueuse,

b) une première fraction de polyssacharides est isolée avec de l'$H_2O$ comme éluant et

c) d'autres polysaccharides, acides, sont élués à l'aide d'un gradient de NaCl de 0 à 0,75 M.

**4.** Procédé, selon la revendication 1 ou 2, caractérisé en ce que les polysaccharides neutres de la précipitation 1 : 1 sont isolés par les étapes de purification suivantes:

a) les polysaccharides sont d'abord soumis à une chromatographie par échange d'anions avec de l'$H_2O$ comme éluant,

b) les fractions purifiées contenant des polysaccharides sont soumises à une autre chromatographie par échange d'anions, éluées avec de l'$H_2O$ et

c) les fractions purifiées de polysaccharides sont encore purifiées par chromatographie sur gel en utilisant de l'$H_2O$ ou du NaCl 0,2M comme éluant.

**5.** Procédé selon la revendication 1 ou 2 caractérisé en ce que les polysaccharides de la précipitation 1 : 4 sont isolés par les étapes de purification suivantes:

a) le polysaccharide neutre est d'abord séparé par chromatographie par échange d'anions en utilisant de l'$H_2O$ comme éluant, ensuite

b) le polysaccharide acide est élué en utilisant un gradient de concentration de NaCl de 0 à 0,4 M,

c) le polysaccharide neutre est encore purifié par chromatographie sur gel en utilisant du NaCl 0,2M ou de l'$H_2O$ comme éluant et

d) le polysaccharide acide est d'abord soumis à une autre chromatographie par échange d'anions en utilisant d'abord l'$H_2O$ pour séparer le polysaccharide neutre accidentellement présent et il est ensuite élué en utilisant un gradient de NaCl de 0 à 0,4 M, et

e) les deux polysaccharides sont soumis à une autre chromatographie par filtration sur gel en utilisant une solution de NaCl 0,2M comme éluant.

**6.** Procédé, selon la revendication 1 ou 2, caractérisé en ce que de l'acétone, du sulfate d'ammonium, du bromure de cétyltriméthylammonium, du $CaCl_2$ ou une solution de Fehling sont utilisés pour la précipitation des polysaccharides.

**7.** Procédé de préparation de polysaccharides, selon une des revendications 1 à 6, caractérisé en ce que

(a) des plantules, particules de feuilles, de fleurs, de tiges et de racines stérilisées de substances végétales de départ d'Echinacea purpurea et Echinacea angustifolia sont utilisées comme substances de départ; celles-ci sont amenées dans un bouillon de culture contenant de l'agar pour la formation de cal, des cellules primaires pour une suspension cellulaire sont extraites selon des méthodes connues à partir du cal formé; les cultures de suspensions cellulaires sont séparées par filtration après 12 à 20, de préférence 14 jours à 22 - 28 °C, les résidus cellulaires sont rejetés,

(b) les troubles résiduels du bouillon de culture causés par des fragments cellulaires ou des dépôts protéinés sont éliminés par clarification dans un séparateur continu, de préférence dans un séparateur à chambre ou à disque et les particules séparées sont éliminées,

(c) les éléments dissous colloïdalement ainsi que les composés de poids moléculaire élevé (poids moléculaire > $10^6$ dalton) sont éliminés par microfiltration, des membranes de polysulfones dans des modules tubulaires ou capillaires avec une dimension des pores ≧ 0,1 µm ou des membranes d'ultrafiltration avec des limites de séparation > $10^5$ dalton étant utilisées,

d) une fois un volume minimal prédéterminé atteint, le rétentat est diafiltré avec de l'eau déminéralisée, le perméat et le diafiltrat sont purifiés, le rétentat est écarté et les perméats purifiés sont concentrés par ultrafiltration (facteurs de concentration entre 10 et 500, en fonction des volumes de départ et de l'importance de l'installation), le tout est ensuite diafiltré avec de l'eau déminéralisée jusqu'à une conductibilité inférieure à 100 µS/cm et le perméat est éliminé,

e) le rétentat concentré est,

e.1. en chauffant à des températures de 90 °C à 130 °C et

e.2. après refroidissement pour la séparation des dépôts, filtré ou centrifugé ou déprotéiné en

e.1. mélangeant la solution avec de l'acide trichloroacétique (TCA) jusqu'à une concentration de 7,5 % en poids,

e.2. la laissant reposer à 4 °C pendant 12 heures au moins,

e.3. séparant par filtration ou en centrifugeant les dépôts,

e.4. en dialysant la solution clarifiée jusqu'à une conductibilité <100 µS/cm à l'eau déminéralisée,

f) le rétentat concentré est d'abord élué sur un échangeur d'anions faible avec de l'eau comme éluant pour extraire la fraction de polysaccharide neutre (correspondant à la fraction de polysaccharide A) et ensuite avec un gradient de sel (acétate de sodium, NaCl, etc.) de 0 à 4 molaire pour l'extraction de la fraction de polysaccharide acide (correspondant à la fraction de polysaccharide F),

g) les fractions individuelles sont éventuellement déprotéinées comme ci-dessus au point e),

h) comme au point d), les fractions individuelles subissent une ultrafiltration et une diafiltration et

i) les produits sont ensuite déshydratés par lyophilisation, séchage sous vide ou séchage par vaporisation.

**8.** Procédé, selon la revendication 7, caractérisé en ce que la déprotéination selon l'étape e) est déjà effectuée après l'étape a).

**9.** Procédé, selon la revendication 7 ou 8, caractérisé en ce que des membranes de polysulfone ou d'acétate de cellulose dans des modules tubulaires, capillaires ou spiralés qui présentent des limites de séparation de 10.000 dalton ou moins sont utilisées à l'étape d).

**10.** Procédé, selon une des revendications 7 à 9, caractérisé en ce que la diafiltration de l'étape d) est remplacée par une déminéralisation par dialyse ou filtration sur gel.

**11.** Procédé de fabrication d'une préparation pharmaceutique caractérisée en ce que les polysaccharides préparés, selon une des revendications 1 à 10, sont mélangés avec des charges et adjuvants classiques compatibles pharmacologiquement.

Figur

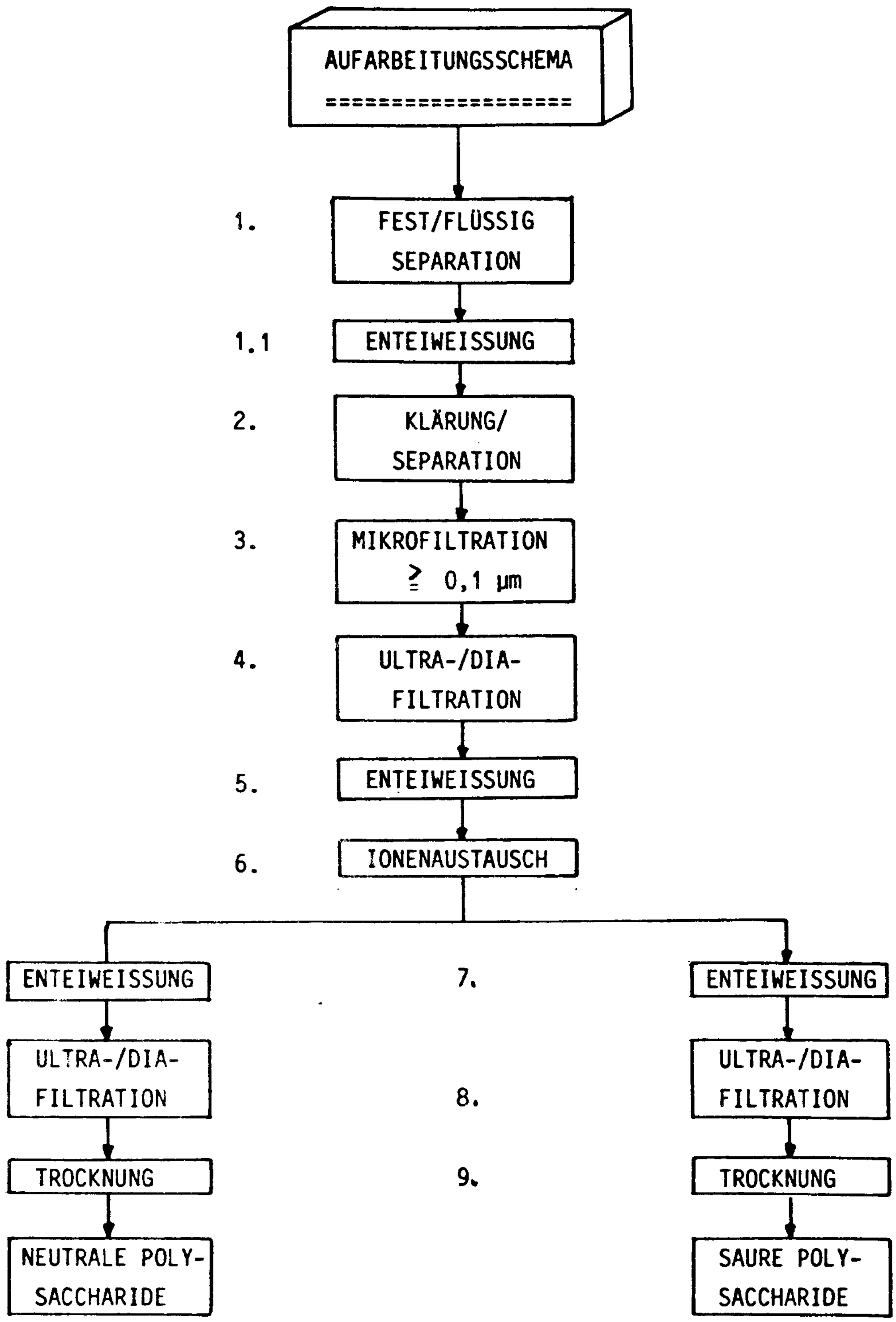
AUFARBEITUNGSSCHEMA
1. FEST/FLÜSSIG SEPARATION
1.1 ENTEIWEISSUNG
2. KLÄRUNG/ SEPARATION
3. MIKROFILTRATION ≧ 0,1 µm
4. ULTRA-/DIA-FILTRATION
5. ENTEIWEISSUNG
6. IONENAUSTAUSCH
7. ENTEIWEISSUNG
8. ULTRA-/DIA-FILTRATION
9. TROCKNUNG
NEUTRALE POLY-SACCHARIDE
SAURE POLY-SACCHARIDE

F I G U R E

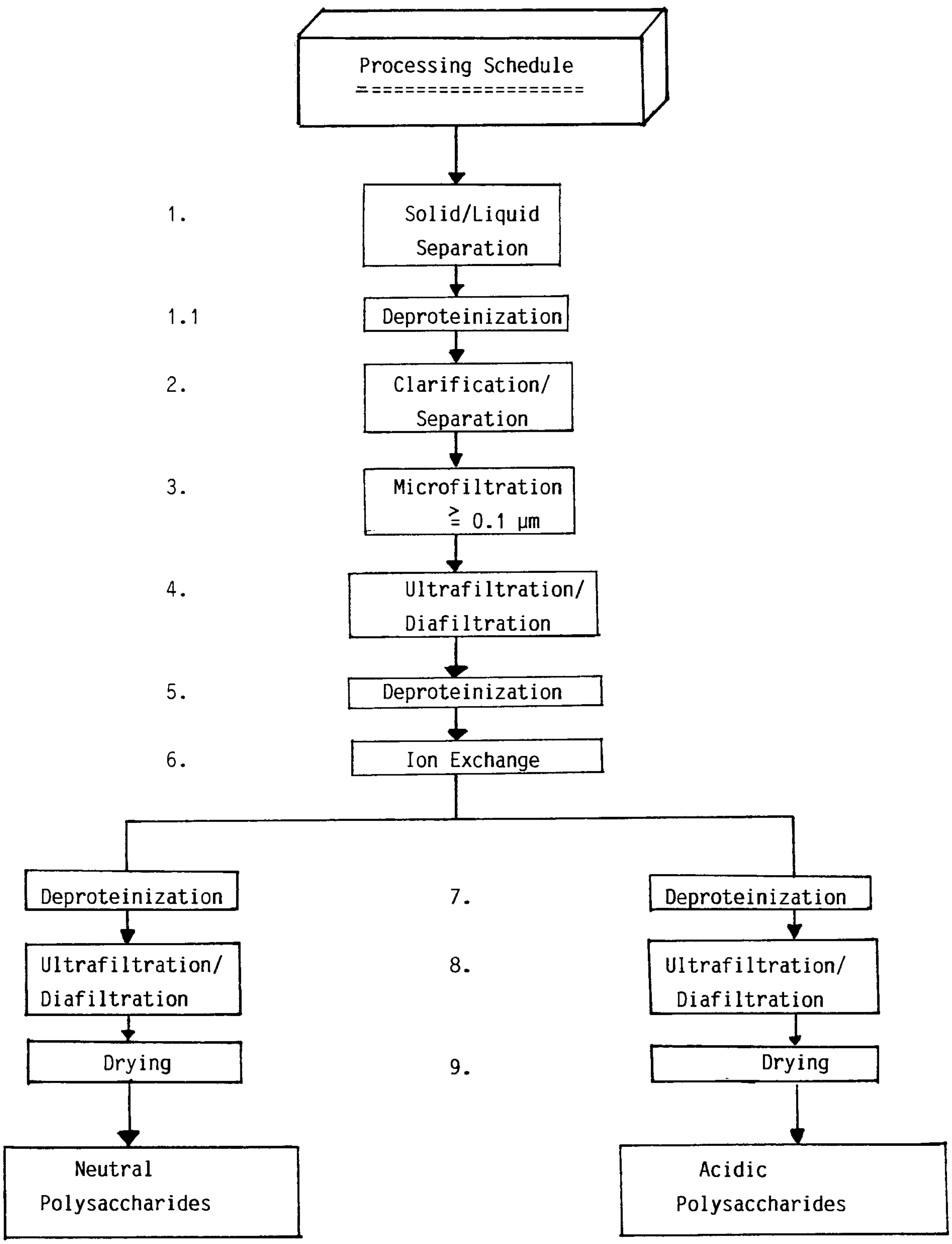
Processing Schedule
1.
Solid/Liquid Separation
1.1
Deproteinization
2.
Clarification/ Separation
3.
Microfiltration ≧ 0.1 µm
4.
Ultrafiltration/ Diafiltration
5.
Deproteinization
6.
Ion Exchange
Deproteinization
7.
Deproteinization
Ultrafiltration/ Diafiltration
8.
Ultrafiltration/ Diafiltration
Drying
9.
Drying
Neutral Polysaccharides
Acidic Polysaccharides

Figure

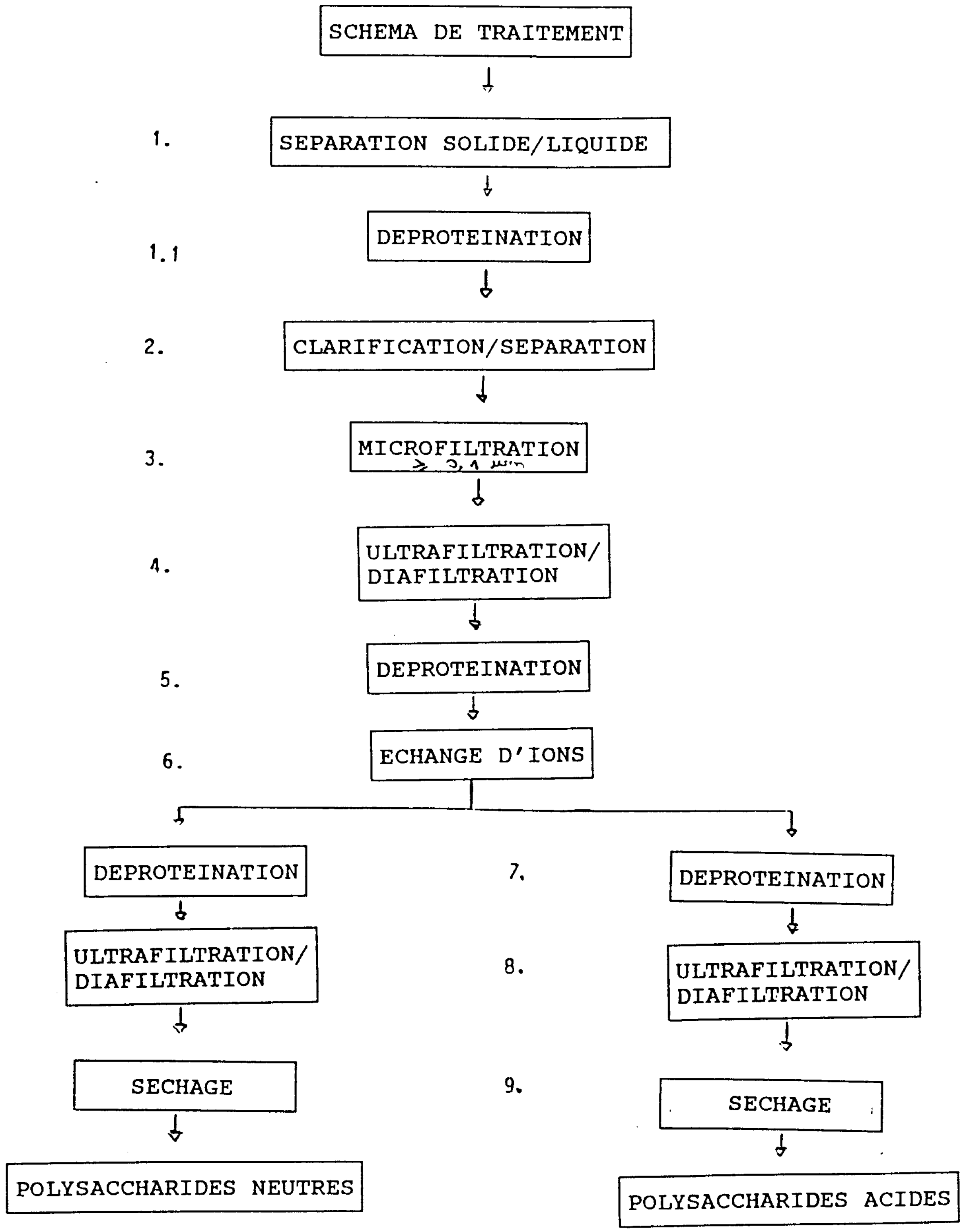
SCHEMA DE TRAITEMENT
1.
SEPARATION SOLIDE/LIQUIDE
1.1
DEPROTEINATION
2.
CLARIFICATION/SEPARATION
3.
MICROFILTRATION
≥ 0,1 µm
4.
ULTRAFILTRATION/
DIAFILTRATION
5.
DEPROTEINATION
6.
ECHANGE D'IONS
7.
DEPROTEINATION
DEPROTEINATION
8.
ULTRAFILTRATION/
DIAFILTRATION
ULTRAFILTRATION/
DIAFILTRATION
9.
SECHAGE
SECHAGE
POLYSACCHARIDES NEUTRES
POLYSACCHARIDES ACIDES